# EUROPEAN PATENT APPLICATION

(11) **EP 3 604 517 A1**
(43) Date of publication of application: **05.02.2020**
(21) Application number: 18775877.6
(22) Date of filing: 30.03.2018
(51) Int. Cl.: C12N 15/06, C07K 16/18, C12N 5/16, C12P 21/08, G01N 33/53, G01N 33/577

(54) **ANTI-APOA1 ANTIBODY**

(30) Priority: 30.03.2017 JP 2017066691
(71) Applicant: Sysmex Corporation, Kobe-shi, Hyogo 651-0073 (JP)
(72) Inventor: MURAKAMI, Katsuhiro, Kobe-shi Hyogo 651-0073 (JP); IINO, Takuya, Kobe-shi Hyogo 651-0073 (JP); MIWA, Keiko, Kobe-shi Hyogo 651-0073 (JP); PENG, Xiaoling, Kobe-shi Hyogo 651-0073 (JP); SAIKI, Naoya, Kobe-shi Hyogo 651-0073 (JP)
(74) Representative: Hoffmann Eitle
(86) International application number: PCT/JP2018/013716
(87) International publication number: WO 2018/181935

(57) **Abstract**

An object of the present invention is to acquire an antibody capable of binding to high-density lipoprotein regardless of the modified state of high-density lipoprotein and whether or not it incorporates labeled cholesterol. Provided is a monoclonal antibody or antigen-binding fragment thereof which binds to a peptide consisting of 51st to 110th amino acid sequence or a peptide consisting of 201st to 267th amino acid sequence of an amino acid sequence of SEQ ID NO: 1, and is capable of binding to high-density lipoproteins of (1) to (3) below;
(1) high-density lipoprotein that is oxidatively modified,
(2) high-density lipoprotein that is not oxidatively modified, and
(3) high-density lipoprotein incorporating labeled cholesterol.

SEQUENCE LISTING

<110>  SYSMEX CORPORATION

<120>  anti-ApoA1 antibody

<130>  P18-038WO

<150>  JP 2017-066691
<151>  2017-03-30

<160>  2

<170>  PatentIn version 3.5

<210>  1
<211>  267
<212>  PRT
<213>  Homo sapiens

<400>  1


<210>  2
<211>  243
<212>  PRT
<213>  Homo sapiens

<400>  2

Asp Glu Pro Pro Gln Ser Pro Trp Asp Arg Val Lys Asp Leu Ala Thr
1               5                   10                  15

Val Tyr Val Asp Val Leu Lys Asp Ser Gly Arg Asp Tyr Val Ser Gln
            20                  25                  30

Phe Glu Gly Ser Ala Leu Gly Lys Gln Leu Asn Leu Lys Leu Leu Asp
        35                  40                  45

Asn Trp Asp Ser Val Thr Ser Thr Phe Ser Lys Leu Arg Glu Gln Leu
    50                  55                  60

Gly Pro Val Thr Gln Glu Phe Trp Asp Asn Leu Glu Lys Glu Thr Glu
65                  70                  75                  80

Gly Leu Arg Gln Glu Met Ser Lys Asp Leu Glu Glu Val Lys Ala Lys
                85                  90                  95

Val Gln Pro Tyr Leu Asp Asp Phe Gln Lys Lys Trp Gln Glu Glu Met
            100                 105                 110

Glu Leu Tyr Arg Gln Lys Val Glu Pro Leu Arg Ala Glu Leu Gln Glu

115                 120                 125

Gly Ala Arg Gln Lys Leu His Glu Leu Gln Glu Lys Leu Ser Pro Leu
    130                 135                 140

Gly Glu Glu Met Arg Asp Arg Ala Arg Ala His Val Asp Ala Leu Arg
145                 150                 155                 160

Thr His Leu Ala Pro Tyr Ser Asp Glu Leu Arg Gln Arg Leu Ala Ala
                165                 170                 175

Arg Leu Glu Ala Leu Lys Glu Asn Gly Gly Ala Arg Leu Ala Glu Tyr
            180                 185                 190

His Ala Lys Ala Thr Glu His Leu Ser Thr Leu Ser Glu Lys Ala Lys
        195                 200                 205

Pro Ala Leu Glu Asp Leu Arg Gln Gly Leu Leu Pro Val Leu Glu Ser
    210                 215                 220

Phe Lys Val Ser Phe Leu Ser Ala Leu Glu Glu Tyr Thr Lys Lys Leu
225                 230                 235                 240

Asn Thr Gln

## Description

### Technical Field

Disclosed herein is a monoclonal antibody or antigen-binding fragment thereof that binds to apolipoprotein A-1 (referred to herein as "ApoA1"). Also disclosed herein are a method for measuring an ability of high-density lipoprotein to uptake cholesterol using the monoclonal antibody or antigen-binding fragment thereof, and a method for quantifying high-density lipoprotein. Furthermore, disclosed herein are a reagent kit for functional evaluation of high-density lipoprotein comprising the monoclonal antibody or antigen-binding fragment thereof, and a kit for quantification of high-density lipoprotein.

### Background Art

In recent years, a function of lipoproteins has attracted attention as an indicator for evaluating the presence of disease and disease risk. For example, high-density lipoprotein (HDL) is one of the lipoproteins, and it is known that an ability of HDL to excrete cholesterol from peripheral tissues is a negative prognostic factor for a risk of cardiovascular disease.

In order to conduct such clinical evaluation correctly, it is important to evaluate the function of lipoproteins quantitatively and accurately. Preferably, after capturing lipoproteins from a living body, it is desirable to remove contaminants by B/F separation and evaluate the function at high S/N. On the other hand, lipoproteins are known to undergo oxidative modification in vivo, and the degree of oxidation is considered to vary depending on individual differences and circumstances of each occasion. In order to correctly perform functional evaluation of lipoproteins, it is desirable to analyze the functions after capturing the lipoproteins regardless of the oxidation state of the lipoproteins.

Non Patent Literature 1 discloses an anti-ApoAl antibody that uniformly binds to HDL regardless of the oxidation state of HDL.

### Citation List

### Patent Literature

Patent Literature 1: US 2016/0,109,469 A

### Non Patent Literature

Non Patent Literature 1: DiDonato JA et al., Circulation. 2013 Oct 8; 128(15): p1644-1655

### Summary of Invention

### Technical Problem

In measuring the function of high-density lipoprotein, labeled cholesterol is often used, and it is required to capture lipoproteins incorporating labeled cholesterol (for example, Patent Literature 1).

An object of the present invention is to provide an antibody and an antigen-binding fragment thereof capable of binding to high-density lipoprotein with or without oxidation and capable of binding not only to high-density lipoprotein not incorporating labeled cholesterol, but also to high-density lipoprotein incorporating labeled cholesterol.

### Solution to Problem

As a result of intensive studies, the present inventors have confirmed that the functional evaluation of high-density lipoprotein can be performed by acquiring a high-density lipoprotein and a monoclonal antibody capable of binding to high-density lipoprotein incorporating labeled cholesterol, regardless of the presence or absence of oxidative modification of the high-density lipoprotein, and using the antibody.

Disclosed herein as a first embodiment of a means for solving the problem is a monoclonal antibody or antigen-binding fragment thereof having
a feature that it is capable of binding to a peptide consisting of 51st to 110th amino acid sequence or a peptide consisting of 201st to 267th amino acid sequence of an amino acid sequence of SEQ ID NO: 1, and
a feature that it is capable of binding to high-density lipoproteins of (1) to (3) below:
(1) high-density lipoprotein that is oxidatively modified,
(2) high-density lipoprotein that is not oxidatively modified, and
(3) high-density lipoprotein incorporating labeled cholesterol represented by (I):

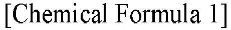

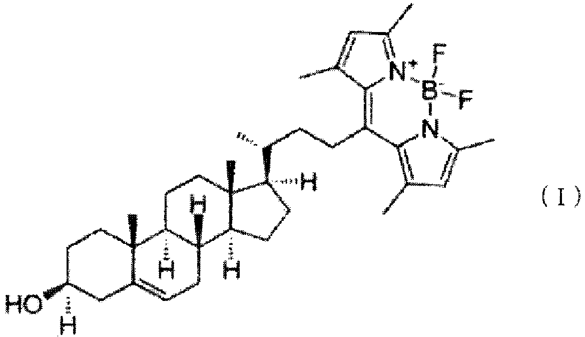

According to the monoclonal antibody or antigen-binding fragment thereof of this embodiment, it can bind to the lipoprotein, regardless of the oxidative modification state of the high-density lipoprotein and the presence or absence of incorporation of labeled cholesterol, thus it is possible to capture the high-density lipoprotein and high-density lipoprotein incorporating labeled cholesterol.

Disclosed herein as a second embodiment of the means for solving the problem is a hybridoma that produces the monoclonal antibody or antigen-binding fragment thereof.

Disclosed herein as a third embodiment of the means for solving the problem is a method for measuring an ability of high-density lipoprotein to uptake cholesterol, including a step of mixing the monoclonal antibody or antigen-binding fragment thereof, labeled cholesterol containing a labeling substance and high-density lipoprotein, thereby forming a complex containing the high-density lipoprotein incorporating the labeling substance and the monoclonal antibody or antigen-binding fragment thereof, and a step of detecting a signal caused by the labeling substance in the complex. According to this embodiment, it is possible to measure the ability to uptake labeled cholesterol for high-density lipoprotein, regardless of the state of oxidative modification.

Disclosed herein as a fourth embodiment of the means for solving the problem is a method for quantifying high-density lipoprotein, including a step of mixing high-density lipoprotein, a capture antibody, and a detection antibody to form a complex, and a step of quantifying the complex. Each of the capture antibody and the detection antibody is one selected from the group consisting of a monoclonal antibody or antigen-binding fragment thereof that binds to 51st to 110th of the amino acid sequence of SEQ ID NO: 1 and a monoclonal antibody or antigen-binding fragment thereof that binds to 201st to 267th of the amino acid sequence of SEQ ID NO: 1. According to this embodiment, it is possible to measure high-density lipoprotein with good reproducibility.

Disclosed herein as a fifth embodiment of the means for solving the problem is a method for measuring an ability of high-density lipoprotein to uptake cholesterol. This embodiment incudes a step of mixing high-density lipoprotein in a first sample acquired from a subject with labeled cholesterol to form high-density lipoprotein incorporating the labeled cholesterol, a step of mixing a first capture antibody and the high-density lipoprotein incorporating the labeled cholesterol, thereby forming a first complex of the high-density lipoprotein incorporating the labeled cholesterol and the capture antibody, and a step of acquiring a first measured value caused by a labeling substance in the first complex; and a step of mixing high-density lipoprotein in a second sample acquired from a same subject as the subject, a second capture antibody, and a detection antibody to form a second complex, a step of acquiring a second measured value of the second complex, and
a step of calculating an ability of high-density lipoprotein to uptake cholesterol, based on the first measured value and the second measured value. In this embodiment, the first capture antibody is the monoclonal antibody or antigen-binding fragment thereof according to any one of claims 1 to 7, and each of the second capture antibody and the detection antibody is a monoclonal antibody or antigen-binding fragment thereof that binds to 51st to 110th of the amino acid sequence of SEQ ID NO: 1 or a monoclonal antibody or antigen-binding fragment thereof that binds to 201st to 267th of the amino acid sequence of SEQ ID NO: 1. According to this embodiment, it is possible to measure the ability to uptake labeled cholesterol with good reproducibility.

The embodiments according to the method of the present invention are performed in vitro.

### Advantageous Effects of Invention

According to the monoclonal antibodies and antigen-binding fragments disclosed herein, it can detect high-density lipoprotein and high-density lipoprotein incorporating labeled cholesterol, regardless of the oxidative modification state of the high-density lipoprotein.

### Brief Description of Drawings

FIG. 1A shows reactivity of each clone to untreated recombinant ApoA1 or oxidized recombinant ApoA1. FIG. 1B shows reactivity of each clone to untreated HDL or oxidized HDL. The horizontal axis shows clone names.
FIG. 2 shows reactivity to untreated HDL or oxidized HDL in a clone different from FIG. 1. The horizontal axis shows clone names.
FIG. 3A shows abilities of 1C5 antibody, 8E10 antibody, P1A5 antibody, and P1A7 antibody to capture labeled cholesterol-HDL FIG. 3B shows abilities of 1C5 antibody, 8E10 antibody, P1A5 antibody, and P1A7 antibody to capture HDL. In FIG. 3, High TG mix. indicates high triglyceride pooled serum, and Normal mix. indicates normal pooled serum.
FIG. 4A shows a comparison result of the abilities of 1C5 antibody to capture labeled cholesterol-HDL between non-oxidized (-) HDL and oxidized (+) HDL. FIG. 4B shows a comparison result of the abilities of 1C5 antibody to capture HDL between non-oxidized (-) HDL and oxidized (+) HDL. In FIG. 4, (-) indicates a negative control. Oxidation (-) indicates non-oxidized, and oxidation (+) indicates oxidized.
FIG. 5 shows binding of 8E10 antibody, P1-A5 antibody, P1-A7 antibody, and 1C5 antibody to a partial sequence contained in the amino acid sequence of SEQ ID NO: 1.
FIG. 6 shows binding of 8E10 antibody and anti-His tag antibody to various partial sequences included in a range of 51st to 110th of the amino acid sequence of SEQ ID NO: 1.
FIG. 7 is a schematic view showing an example of a reagent kit for functional evaluation of high-density lipoprotein.
FIG. 8 is a schematic view showing an example of a reagent kit for quantification of high-density lipoprotein.

### Description of Embodiments

### [Monoclonal antibody or antigen-binding fragment thereof]

The first embodiment relates to a monoclonal antibody or antigen binding fragment thereof that binds to ApoA1.

ApoA1 means apolipoprotein A-1, and is preferably human-derived ApoA1. Further preferably, it is a protein shown in SEQ ID NO: 1. In vivo, ApoA1 of SEQ ID NO: 1 is transcribed and translated from the ApoA1 gene and then cleaved at an N-terminal side. It is present as a mature ApoA1 shown in SEQ ID NO: 2 in HDL. In this embodiment, when referring to a specific region on the amino acid sequence of ApoA1, it is based on the amino acid sequence set forth in SEQ ID NO: 1. Also, in this embodiment, ApoA1 contains proteins having a homology of 70% or more, preferably 80% or more, more preferably 85% or more, further preferably 90% or more, and particularly preferably 95% or more, with the amino acid sequence of SEQ ID NO: 1. The homology can be determined using blastp (https://blast.ncbi.nlm.nih.gov/Blast.cgi?PAGE=Proteins&PROGRAM=blastp&BLAST _PROGRAMS=blastp&PAGE_TYPE=BlastSearch&DBSEARCH=true&QUERY=&S UBJECTS=) published in National Center for Biotechnology Information (NCBI), and the like.

The monoclonal antibody binds to a range of 51st to 110th or the range of 201st to 267th of the amino acid sequence of SEQ ID NO: 1. Preferably, the monoclonal antibody binds to a range of 91st to 110th of the amino acid sequence of SEQ ID NO: 1. Moreover, it is preferable that the monoclonal antibody of this embodiment does not bind to a range of 101st to 160th of the amino acid sequence of SEQ ID NO: 1. Further, it is preferable that the monoclonal antibody of this embodiment also does not bind to ranges of 2nd to 60th and 151st to 210th of the amino acid sequence of SEQ ID NO: 1.

Furthermore, the monoclonal antibody can bind to:
(1) high-density lipoprotein that is oxidatively modified,
(2) high-density lipoprotein that is not oxidatively modified, and
(3) high-density lipoprotein incorporating labeled cholesterol represented by formula (I):

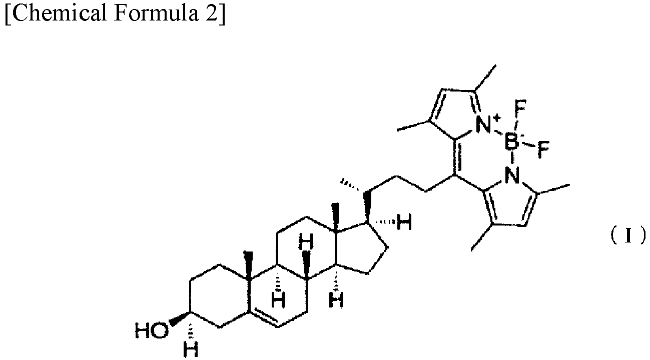

Here, high-density lipoprotein is also referred to as HDL. The high-density lipoprotein is a lipoprotein having a density of 1.063 g/mL or more.

The oxidative modification of high-density lipoprotein means oxidation of at least ApoA1 protein or lipid contained in the high-density lipoprotein, and is not particularly limited in this regard. Examples of oxidative modification of ApoA1 can include malondialdehyde modification; chlorination of 192nd tyrosine residue of mature ApoA1, nitration of 18th tyrosine residue (J Biol Chem. 2012 Feb 24; 287(9):6375-86.), and hydroxylation of 72nd tryptophan residue (Nat Med. 2014 Feb; 20(2): 193-203.). Examples of oxidative modification of lipid can include oxidized phosphatidylcholine modification (J. Biol. Chem. 2, 69, 15274-15279, 1994) and the like. Examples of a method for performing oxidative modification in vitro can include a method of oxidizing high-density lipoprotein using hydrogen peroxide or the like, as described in Non Patent Literature 1.

The phrase "bind to high-density lipoprotein" is not limited as long as the monoclonal antibody or antigen-binding fragment thereof binds to at least a part of ApoA1 contained in high-density lipoprotein by an antigen-antibody reaction. The term "binding" preferably means that it can capture high-density lipoprotein. More preferably, binding refers to binding that occurs in PBS without bovine serum albumin or StartingBlock (PBS) Blocking buffer (Thermo Fisher SCIENTIFIC/Product No. 37538) without bovine serum albumin. The term "bind" refers that when the monoclonal antibody or antigen-binding fragment thereof is used as a capture antibody to detect high-density lipoprotein or a peptide having the amino acid sequence of SEQ ID NO: 1 by ELISA, a signal of a well containing the high-density lipoprotein or the peptide having the amino acid sequence is higher than a signal of a well containing a negative control such as PBS. The phrase "does not bind" refers that when the monoclonal antibody or antigen-binding fragment thereof is used as a capture antibody to detect high-density lipoprotein or a peptide having the amino acid sequence of SEQ ID NO: 1 by ELISA, a signal of a well containing the high-density lipoprotein or the peptide having the amino acid sequence is equivalent or lower than a signal of a well containing a negative control such as PBS. The binding is not limited to that generated to non-denatured high-density lipoprotein, but may be generated to denatured high-density lipoprotein. Preferably, the monoclonal antibody or antigen-binding fragment thereof binds to high-density lipoprotein present in a non-denatured state in a sample under non-denaturing conditions. The non-denatured high-density lipoprotein refers, for example, that a complex of protein and lipid is maintained. Denaturation, for example, refers that the complex of protein and lipid is not maintained. In addition, denaturation may include a state in which all or part of higher order structures of secondary or higher structures of the protein is reversibly or irreversibly destroyed. Specifically, denaturation include, for example, a state in which all or part of charges of high-density lipoprotein is changed, and/or all or part of disulfide bonds of proteins contained in high-density lipoprotein is cleaved.

The sample is not particularly limited as long as it contains high-density lipoprotein derived from a specimen acquired from a subject such as a mammal, preferably human high-density lipoprotein. Examples of the specimen include blood samples such as blood, serum and plasma. The sample may be, for example, a specimen itself, may be a specimen diluted with physiological saline, PBS or the like, may be a suspension of high-density lipoprotein recovered from the specimen by polyethylene glycol method described in Patent Literature 1, or the like. The sample preferably contains a fatty acid that forms an ester with cholesterol.

In addition, examples of the monoclonal antibody of the first embodiment can include a monoclonal antibody produced from a hybridoma of accession No. NITE BP-02442 or accession No. NITE BP-02443 or its progeny (including a passage). The hybridomas identified by accession No. NITE BP-02442 and accession No. NITE BP-02443 have been internationally deposited, as identification references "8E10" and "PIA5", respectively, at the Patent Microorganisms Depositary of the National Institute of Technology and Evaluation, located in Room 122, 2-5-8, Kazusakamatari, Kisarazu-shi, Chiba 292-0818 Japan, on March 8, 2017. Herein, antibodies produced from these hybridomas may be referred to as 8E10 antibody and P1A5 antibody, respectively. The monoclonal antibody can bind all the high-density lipoproteins of (1) to (3) in the feature.

The monoclonal antibody of the first embodiment also includes a monoclonal antibody containing a same amino acid sequence as all or part of the monoclonal antibodies produced from the above hybridomas or their progeny, as far as they have the above features.

Moreover, the monoclonal antibody includes a monoclonal antibody of which amino acid sequences of their heavy chain complementarity chain determining region (HCDR) and light chain complementarity determining region (LCDR) are the same amino acid sequences of HCDR and LCDR of each monoclonal antibody produced from the hybridoma of accession No. NITE BP-02442 or accession No. NITE BP-02443 or its progeny. Specifically, the monoclonal antibody includes a monoclonal antibody having HCDR1, HCDR2, HCDR3, LCDR1, LCDR2 and LCDR3 comprising the amino acid sequences identical to the amino acid sequences of HCDR1, HCDR2, HCDR3, LCDR1, LCDR2 and LCDR3 of each monoclonal antibody produced from the hybridoma of accession No. NITE BP-02442 or accession No. NITE BP-02443 or its progeny.

In general, an antigen-binding fragment means a part of an antibody that retains an ability to bind antigen and/or an ability to recognize antigen. In this embodiment, the antigen-binding fragment is a part of the above-described monoclonal antibody, and has features (a) to (e) by retaining an ability to bind its antigen ApoA1 and/or an ability to recognize antigen as in the case of the monoclonal antibody.
(a) It binds to a peptide consisting of 51st to 110th amino acid sequence, preferably 91st to 110th amino acid sequence of the amino acid sequence of SEQ ID NO: 1, or
(b) it binds to a peptide consisting of 201st to 267th amino acid sequence of the amino acid sequence of SEQ ID NO: 1.

Preferably, (c) it binds to a peptide consisting of 91st to 110th amino acid sequence of the amino acid sequence of SEQ ID NO: 1.

More preferably, (d) it does not bind to a range of 101st to 160th of the amino acid sequence of SEQ ID NO: 1.

Further preferably, (e) it does not bind to a peptide consisting of 2nd to 60th and 151st to 210th amino acid sequences of the amino acid sequence of SEQ ID NO: 1.

Furthermore, in addition to the features (a) to (e) above, the antigen-binding fragment has a feature that it is capable of binding to:
(1) high-density lipoprotein that is oxidatively modified,
(2) high-density lipoprotein that is not oxidatively modified, and
(3) high-density lipoprotein incorporating labeled cholesterol represented by the formula (I):

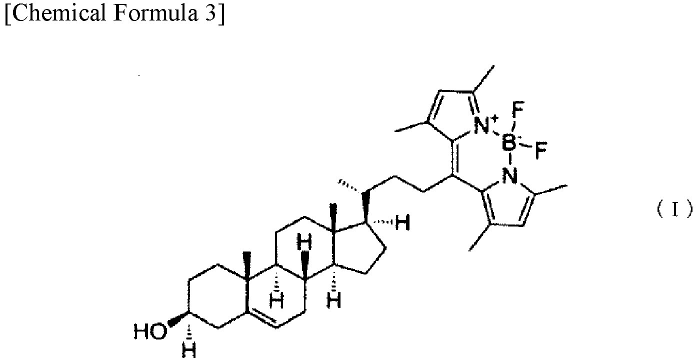

The antigen-binding fragment targeted by this embodiment may be one having the features, and is not limited, and may be, for example, a peptide containing Fab, Fab', F(ab')2, scFv, diabody, dsFv or CDR of the above-described monoclonal antibody, or the like. A method for preparing these antigen-binding fragments is known, and the antigen-binding fragment can be prepared, for example, by a method described in US 2016/0,159,895 A.

### [Hybridoma]

A second embodiment relates to a hybridoma identified by accession No. NITE BP-02442 or accession No. NITE BP-02443 or its progeny. The progeny includes deposited hybridoma itself, those obtained by culturing the deposited hybridoma, those distributed from the deposited hybridoma, further passage cells thereof, and the like. The hybridoma has been internationally deposited at the international depository authority on March 8, 2017, as described above. An example of a method for preparing a hybridoma is shown in Example 1 described later.

### [Method 1 for measuring ability of high-density lipoprotein to uptake cholesterol]

The third embodiment relates to a method for measuring an ability of high-density lipoprotein to uptake cholesterol (hereinafter sometimes referred to simply as a measuring method).

### <Formation of complex>

In the third embodiment, the monoclonal or antigen-binding fragment described in the section of [Monoclonal antibody or antigen-binding fragment thereof] above and high-density lipoprotein incorporating labeled cholesterol are mixed, thereby forming a complex (also referred to as a first complex) of the high-density lipoprotein incorporating the labeled cholesterol and the monoclonal antibody or antigen-binding fragment thereof. Preferably, one of the monoclonal antibody or antigen-binding fragment thereof that forms a complex with the high-density lipoprotein incorporating the labeled cholesterol preferably may be a capture antibody (also referred to as a first capture antibody) for capturing the high-density lipoprotein incorporating the labeled cholesterol into a solid phase described later or the like.

The high-density lipoprotein is preferably contained in a sample. For the sample, the description in the section of [Monoclonal antibody or antigen-binding fragment thereof] above is incorporated herein.

The conditions for mixing the monoclonal antibody or antigen-binding fragment with the high-density lipoprotein incorporating the labeled cholesterol are not limited as long as the monoclonal antibody or antigen-binding fragment can bind to the high-density lipoprotein incorporating the labeled cholesterol.

The capture antibody may be in a state bound to a solid phase such as a magnetic bead, a plastic bead or a plate when mixed with the high-density lipoprotein incorporating the labeled cholesterol. Immobilization of the monoclonal antibody or antigen-binding fragment can be performed according to known methods.

When the capture antibody is immobilized to a solid phase, the mode of immobilization of the capture antibody on the solid phase is not particularly limited. For example, the capture antibody and the solid phase may be bound directly, or the capture antibody and the solid phase may be indirectly bound via another substance. Examples of the direct binding include physical adsorption and the like. Examples of the indirect binding include a bond via a combination with avidins. In this case, by modifying the capture antibody with biotin in advance and previously binding avidins to the solid phase, the capture antibody and the solid phase can be indirectly bound via a bond between the biotin and the avidins. In the present embodiment, it is preferable that the bond between the capture antibody and the solid phase is an indirect bond via biotin and avidins.

The material of the solid phase is not particularly limited, and it can be selected from, for example, organic polymer compounds, inorganic compounds, biopolymers, and the like. Examples of the organic polymer compound include latex, polystyrene, polypropylene, and the like. Examples of the inorganic compound include magnetic bodies (iron oxide, chromium oxide, ferrite, etc.), silica, alumina, glass, and the like. Examples of the biopolymer include insoluble agarose, insoluble dextran, gelatin, cellulose, and the like. Two or more of these may be used in combination. The shape of the solid phase is not particularly limited, and examples thereof include particles, membranes, microplates, microtubes, test tubes, and the like. Among them, particles are preferable, and magnetic particles are particularly preferable.

Labeled cholesterol is a substance in which a molecule to be labeled (labeling substance) is bound to a part of cholesterol molecule. Here, a cholesterol moiety in the labeled cholesterol may have a structure of naturally occurring cholesterol, or a structure of cholesterol obtained by removing one or more methylene groups and/or methyl groups from an alkyl chain bonded to C17 position of naturally occurring cholesterol (also called norcholesterol).

It is more preferable to use labeled cholesterol which is esterified by lipoproteins, since lipoproteins incorporate cholesterol by esterification. In the method of this embodiment, the labeled cholesterol can be esterified with the fatty acid contained in the sample by an activity of lecithin-cholesterol acyltransferase (LCAT) contained in the high-density lipoprotein when mixed with the high-density lipoprotein. Methods for confirming esterification of labeled cholesterol by lipoproteins themselves are known in the art and can be routinely performed by those skilled in the art.

The labeling substance is not particularly limited as long as a detectable signal is generated. For example, it may be a substance which itself generates a signal (hereinafter also referred to as "signal generating substance") or a substance which catalyzes the reaction of other substances to generate a signal. Examples of the signal generating substance include fluorescent substances, luminescent substances, color-developing substances, radioactive isotopes, and the like.

Labeling of the labeling substance to the antibody can be performed by a labeling method known in the field of immunoassay technology. Labeling may also be performed using a commercially available labeling kit or the like.

Preferably, the fluorescent substance contains a fluorophore having a polar structure. In the art, labeled cholesterol containing fluorescently labeled cholesterol and a fluorophore having a polar structure itself is known.

Examples of the fluorescently labeled cholesterol containing a fluorophore having a polar structure include fluorescently labeled cholesterol containing a fluorophore having a boron dipyrromethene (BODIPY (registered trademark)) backbone structure represented by formula (II): 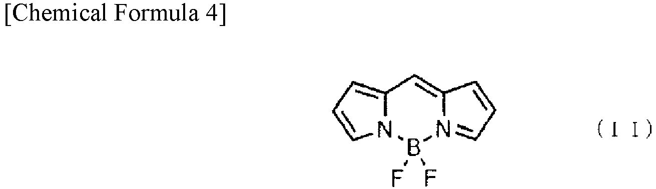 a benzoxadiazole skeleton structure represented by formula (III): 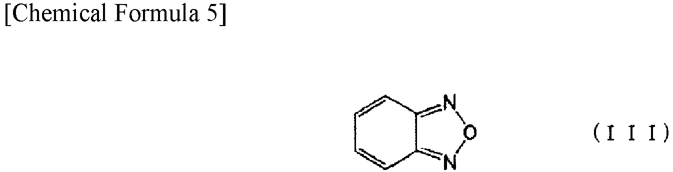 , or the like.

The labeled cholesterol is preferably a compound represented by formula (I):

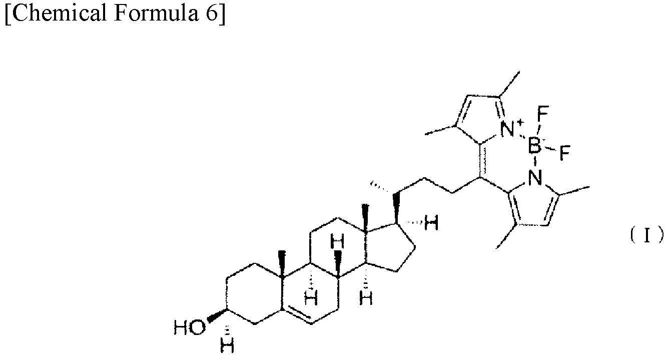

((23-(dipyrrometheneboron difluoride)-24-norcholesterol, trade name TopFluor Cholesterol, CAS No: 878557-19-8, available from Avanti Polar Lipids, Inc.), or
a compound represented by formula (IV):

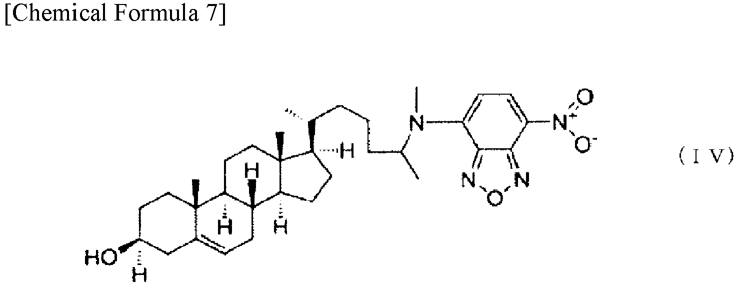

(25-[N-[(7-nitro-2-1,3-benzoxadiazaol-4-yl)methyl]amino]-27-norcholesterol, trade name 25-NBD Cholesterol, CAS No: 105539-27-3, available from Avanti Polar Lipids, Inc.),
and more preferably a compound represented by the formula (I).

As the fluorescent substance, in addition to the above, fluorescent dyes such as fluorescein isothiocyanate (FITC), rhodamine and Alexa Fluor (registered trademark), fluorescent proteins such as Enhanced green fluorescent protein (EGFP) or the like can be used.

As the radioactive isotope, ¹²⁵I, ¹⁴C, ³²P or the like can be used.

Examples of the substance that catalyzes the reaction of other substances to generate a detectable signal include enzymes. Examples of the enzymes include alkaline phosphatase (ALP), peroxidase, β-galactosidase, luciferase, and the like.

The labeled cholesterol may have a tag as a labeling substance. Cholesterol labeled with tag is also called tagged cholesterol. In the case of using tagged cholesterol, a signal can be detected using a substance that specifically binds to the tag (hereinafter, "capture body") in the step of signal detection described later. The tag may be any of naturally occurring substances and synthesized substances, and examples thereof include compounds, peptides, proteins, nucleic acids, combinations thereof, and the like. The compound may be a labeling compound known in the art as long as a substance capable of specifically binding to the compound is present or obtained, and examples thereof include dye compounds and the like.

It is known in the art that cholesterol is esterified to increase its lipid solubility and promote its uptake by lipoproteins. The tag attached to cholesterol may be a lipid soluble or hydrophobic substance.

Examples of combinations of a tag and a substance capable of specifically binding to the tag include an antigen and an antibody that recognizes the antigen, a hapten and an anti-hapten antibody, a peptide or a protein and an aptamer that recognizes them, a ligand and a receptor thereof, oligonucleotides and oligonucleotides having complementary strands thereof, biotin and avidin (or streptavidin), histidine tag (a peptide containing histidine of 6 to 10 residues) and nickel, glutathione-S-transferase (GST) and glutathione, and the like. The antigen as a tag may be a peptide tag and a protein tag known in the art, and examples thereof include FLAG (registered trademark), hemagglutinin (HA), Myc protein, green fluorescent protein (GFP), and the like. Examples of the hapten as a tag include 2,4-dinitrophenol and the like.

The antibody used as a capture body may be a polyclonal antibody, a monoclonal antibody, and a fragment thereof (for example, Fab, F(ab)2, and the like) obtained by immunizing nonhuman animals using a labeling substance or a part thereof as an antigen. Also, immunoglobulin classes and subclasses are not particularly limited.

An example of the tag includes a tag having a structure represented by the formula (II) or a structure represented by formula (V):

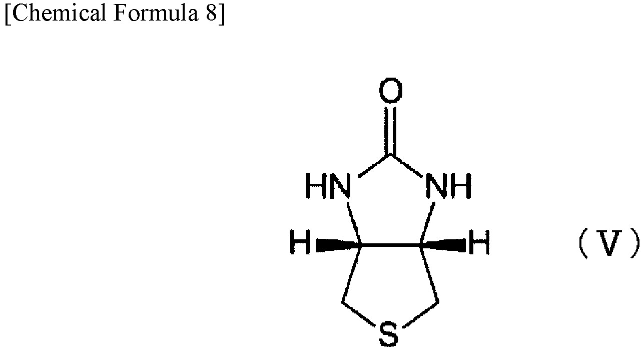

The structure represented by the formula (II) is a boron dipyrromethene (BODIPY (registered trademark)) backbone, and the structure represented by the formula (V) shows a part of biotin. Tagged cholesterol containing a structure represented by the formula (II) or (V) is preferable since capture bodies for these tags are generally available.

Also, cholesterol to which a tag having a 2,4-dinitrophenyl (DNP) group is added is also preferable because anti-DNP antibody is commercially available.

Examples of the tagged cholesterol include tagged cholesterol represented by formula (VI): 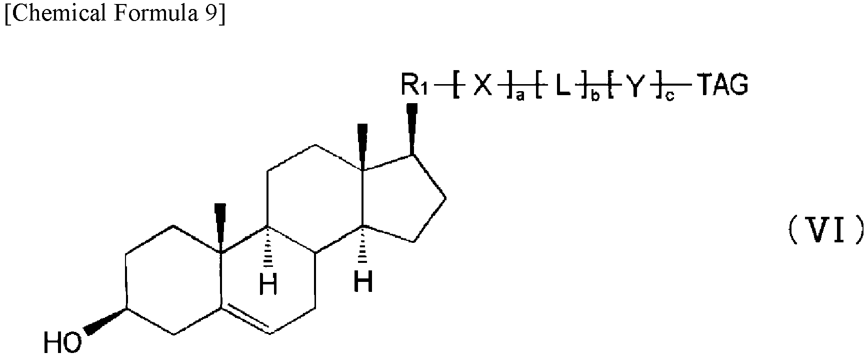
wherein R₁ is an alkylene group having 1 to 6 carbon atoms which may have a methyl group,
X and Y are identical or different, and represent -R₂-NH-, -NH-R₂-, -R₂-(C=O)-NH-, -(C=O)-NH-R₂-, -R₂-NH-(C=O)-, -NH-(C=O)-R₂-, -R₂-(C=O)-, -(C=O)-R₂-, -R₂-(C=O)-O-, -(C=O)-O-R₂-, -R₂-O-(C=O)-, -O-(C=O)-R₂-, -R₂-(C=S)-NH-, - (C=S)-NH-R₂-, -R₂-NH-(C=S)-, -NH-(C=S)-R₂-, -R₂-O-, -O-R₂-, -R₂-S-, or -S-R₂-, wherein each R₂ is independently an atomic bonding, an alkylene group having 1 to 10 carbon atoms which may have a substituent, or an arylene group or heteroarylene group having 6 to 12 carbon atoms which may have a substituent, or a cycloalkylene group or heterocycloalkylene group having 3 to 8 carbon atoms which may have a substituent;
L represents -(CH₂)_{d}-[R₃-(CH₂)ₑ]_{f}- or -[(CH₂)ₑ-R₃]_{f}-(CH₂)_{d}-, and R₃ is an oxygen atom, a sulfur atom, -NH-, -NH-(C=O)- or -(C=O)-NH-;
TAG is a tag;
a and c are identical or different and are an integer of 0 to 6,
b is 0 or 1;
d and e are identical or different and are an integer of 0 to 12; and
f is an integer of 0 to 24.

In the formula (VI), when a, b and c are all 0, the tagged cholesterol represented by the formula has no linker, and the tag and the cholesterol moiety are bonded directly. In the formula (VI), when any one of a, b and c is not 0, the tagged cholesterol represented by the formula has a linker (-[X]ₐ-[L]_{b}-[Y]_{c}-) between the tag and the cholesterol moiety. It is believed that the linker further facilitates the bonding between the tag exposed on the outer surface of the lipoprotein and the capture body. Each substituent in the formula (VI) will be described below.

R₁ may have an alkylene group having 1 to 6 carbon atoms as a main chain, and may have a methyl group at any position. R₁ corresponds to an alkyl chain bonded to C17 position of naturally occurring cholesterol. In this embodiment, when R₁ has 1 to 5 carbon atoms, R₁ preferably has a methyl group at the position of C20 in the naturally occurring cholesterol. When R₁ has 6 carbon atoms, R₁ preferably has the same structures as alkyl chains at C20 to C27 positions of the naturally occurring cholesterol.

[X]ₐ corresponds to a connecting moiety between R₁ and L, [Y]_{c} or the tag. [Y]_{c} corresponds to a connecting moiety between R₁, [X]ₐ or L and the tag. X and Y are determined according to the type of reaction that bonds between the cholesterol moiety and the linker and the type of reaction that bonds between the linker and the tag.

In R₂, the atomic bonding refers to a direct bonding without intervening any other atom. When R₂ is an alkylene group having 1 to 10 carbon atoms, examples of the alkylene group include methylene, ethylene, propylene, isopropylene, butylene, isobutylene, pentylene, neopentylene, hexylene, heptylene, octylene, 2-ethylhexylene, nonylene, and decylene group. Among them, an alkylene group having 1 to 4 carbon atoms is preferred. When R₂ is an alkylene group having a substituent, the above number of carbon atoms does not include the number of carbon atoms in the substituent.

When R₂ is an arylene group or a heteroarylene group, the group may be an aromatic ring having 6 to 12 carbon atoms which may contain one or more hetero atoms selected from N, S, O, and P. Examples of the group include phenylene, naphthylene, biphenylylene, furanylene, pyrrolene, thiophenylene, triazolene, oxadiazolene, pyridylene, and pyrimidylene group. When R₂ is an arylene group or a heteroarylene group having a substituent, the above number of carbon atoms does not include the number of carbon atoms in the substituent.

When R₂ is a cycloalkylene group or a heterocycloalkylene group, the group should be a non-aromatic ring having 3 to 8 carbon atoms which may include one or more hetero atoms selected from N, S, O, and P. Examples of the group include cyclopropylene, cyclobutylene, cyclopentylene, cyclohexylene, cycloheptylene, cyclooctylene, pyrrolidinylene, piperidinylene, and morpholinylene group. When R₂ is a cycloalkylene group or a heterocycloalkylene group having a substituent, the above number of carbon atoms does not include the number of carbon atoms in the substituent.

Examples of the substituent in R₂ include hydroxyl, cyano, alkoxy, =O, =S, - NO₂, -SH, halogen, haloalkyl, heteroalkyl, carboxyalkyl, amine, amide, and thioether group. R₂ may have a plurality of the substituents. Here, halogen represents fluorine, chlorine, bromine, or iodine. Alkoxy represents an -O-alkyl group, and the alkyl group is a linear or branched saturated aliphatic hydrocarbon group having 1 to 5 carbon atoms, and preferably 1 or 2 carbon atoms.

Preferably, a and c are both 1, and X and Y are identical or different and are - (C=O)-NH- or -NH-(C=O)-.

L corresponds to a spacer and has a polymer structure that adds a predetermined length to the linker. It is preferable that the polymer structural moiety does not inhibit the uptake of cholesterol by lipoproteins, and that the linker moiety is hardly incorporated into lipoproteins. The polymers include hydrophilic polymers such as polyethylene glycol (PEG). In a preferred embodiment, L is a structure represented by -(CH₂)_{d}-[O-(CH₂)ₑ]_{f}- or -[(CH₂)ₑ-O]_{f}-(CH₂)_{d}-. Here, d and e are identical or different and are an integer of 0 to 12, preferably an integer of 2 to 6, and more preferably both 2. f is an integer of 0 to 24, preferably an integer of 2 to 11, and more preferably an integer of 4 to 11.

Example of the tagged cholesterol without the linker includes a fluorescently labeled cholesterol (23-(dipyrromethene boron difluoride)-24-norcholesterol) represented by the formula (I). In the tagged cholesterol, the tag (the fluorescent moiety having BODIPY backbone structure) is directly bonded at C23 position of cholesterol. As a capture body that specifically binds to the fluorescent moiety having BODIPY backbone structure, an anti-BODIPY antibody (BODIPY FL Rabbit IgG Fraction, A-5770, Life technologies Corporation) is commercially available.

The tagged cholesterol in which the tag is bound via a linker includes a biotin-tagged cholesterol represented by formula (VII): 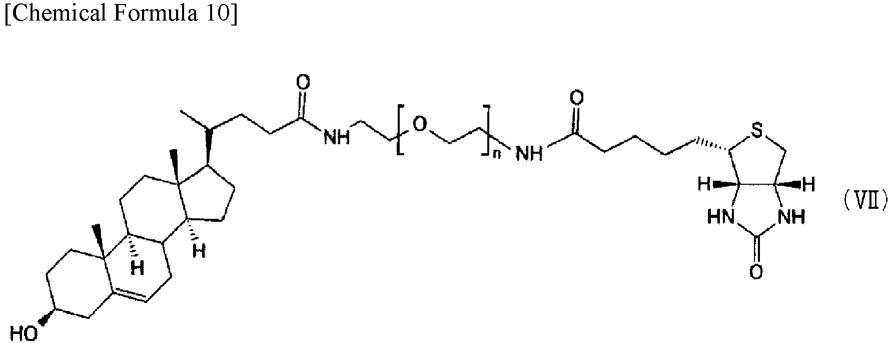 wherein, n is an integer of 0 to 24, preferably an integer of 2 to 11, and more preferably an integer of 4 to 11. In the tagged cholesterol, the tag (the biotin moiety represented by the formula (V)) is bonded to the cholesterol moiety via the linker (polyethylene glycol). As a capture body that specifically binds to the biotin moiety, avidin or streptavidin is suitable. In addition, avidin or streptavidin to which a labeling substance such as horseradish peroxidase (HRP) or alkaline phosphatase (ALP) is bound is also commercially available.

Also, the tagged cholesterol in which the tag is bound via a linker includes DNP-added cholesterol represented by formula (VIII): 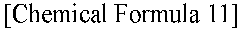 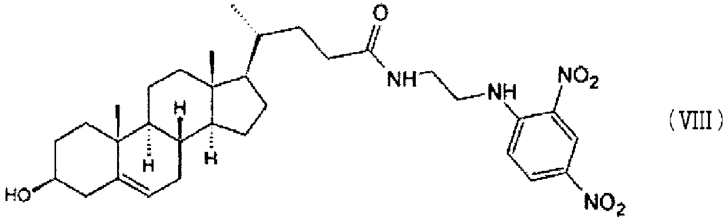 In the tagged cholesterol, DNP is bound to the cholesterol moiety via a linker (-(C=O)-NH-CH₂-CH₂-NH-).

As a capture body that specifically binds to DNP, an anti-DNP antibody is suitable. In addition, anti-DNP antibodies to which a labeling substance such as HRP or ALP is bound is also commercially available.

Although the bonding form of the cholesterol moiety and the tag is not particularly limited, the cholesterol moiety and the tag may be bonded, or the cholesterol moiety and the tag may be bonded via a linker. Although the bonding means is not particularly limited, for example, a crosslink by utilizing a functional group is preferred because it is convenient. Although the functional group is not particularly limited, an amino group, a carboxyl group and a sulfhydryl group are preferred because commercially available crosslinkers can be used.

Since cholesterol has no functional group in the alkyl chain bonded to the C17 position, a cholesterol derivative having a functional group in the alkyl chain is preferably used in the addition of the tag. Examples of the cholesterol derivative include precursors of bile acid, precursor of steroids, and the like. Specifically, 3β-hydroxy-A5-cholenoic acid, 24-amino-5-colen-3β-ol and the like are preferable. The functional group of the tag varies depending on the type of the tag. For example, when a peptide or a protein is used as the tag, an amino group, a carboxyl group and a sulfhydryl group (SH group) can be used. When biotin is used as the tag, a carboxyl group in the side chain can be used. The linker is preferably a polymer compound having functional groups at both terminals. When biotin is added as the tag, a commercially available biotin labeling reagent may be used. The reagent contains biotin to which various length of spacer arms (for example, PEG) having a functional group such as an amino group are bound at the terminal.

Bound/Free (B/F) separation for removing unreacted free components not forming a complex may be carried out after formation of the complex, preferably after formation of the complex and before detection of a labeling substance. The unreacted free component refers to a component not constituting a complex. Examples thereof include components other than high-density lipoprotein in the sample. The means of B/F separation is not particularly limited, and when the solid phase is a particle, B/F separation can be performed by recovering only the solid phase capturing the complex by centrifugation. When the solid phase is a container such as a microplate or a microtube, B/F separation can be performed by removing a liquid containing an unreacted free component. When the solid phase is a magnetic particle, B/F separation can be performed by aspirating and removing a liquid containing an unreacted free component by a nozzle while magnetically constraining the magnetic particles with a magnet. This method is preferable from the viewpoint of automation. After removing the unreacted free component, the solid phase capturing the complex may be washed with a suitable aqueous solvent such as phosphate buffered saline (PBS).

In the measuring method according to the present embodiment, the complex formed as described above is labeled by bonding a capture body that specifically binds to the above-described tag and a labeling substance.

The capture body that specifically binds to the tag may be appropriately determined according to the type of the tag of the tagged cholesterol. For example, with reference to a combination of the tag and the material which can specifically bind to the tag, the capture body can be selected from an antibody, a ligand receptor, an oligonucleotide, biotin, avidin (or streptavidin), a histidine tag or nickel, GST, glutathione and the like. Among them, as the capture body, an antibody that specifically binds to the tag is preferred. The antibody may be a commercially available antibody, or an antibody prepared by a method known in the art. The antibody may be a monoclonal antibody or a polyclonal antibody. The origin and isotype of the antibody are not particularly limited. Also, fragments of the antibody and derivatives thereof can be used, and examples thereof include Fab fragments, F(ab')2 fragments, and the like.

As the labeling substance, a signal generating substance, a substance that catalyzes a reaction of other substances to generate a detectable signal, or the like can be used. Examples of the signal generating substance are fluorescent substances, radioactive isotopes, and the like. Examples of the substance that catalyzes the reaction of other substances to generate a detectable signal are enzymes. The enzymes include peroxidase, alkaline phosphatase, β-galactosidase, glucose oxidase, tyrosinase, acid phosphatase, luciferase, and the like. Examples of the fluorescent substance include fluorescent dyes such as fluorescein isothiocyanate (FITC), rhodamine and Alexa Fluor (registered trademark), fluorescent proteins such as GFP, and the like. Examples of the radioactive isotopes include ¹²⁵I, ¹⁴C, ³²P, and the like. Among them, an enzyme is preferable as the labeling substance.

In a preferred embodiment, the complex is labeled by indirectly bonding the labeling substance to the complex via the capture body that specifically binds to the tag. Examples include bonding the capture body bound to the labeling substance to the complex, or bonding the labeling substance to the capture body bound to the complex. The capture body to which the labeling substance was previously bound may be used, or the labeling substance that can specifically bind to the capture body may be used.

The capture body to which the labeling substance was previously bound can be obtained, for example, by labeling the capture body that specifically binds to the tag with the labeling substance. Here, methods for labeling a substance themselves are known in the art. When the capture body is an antibody, the capture body may be labeled using a commercially available labeling kit or the like. Examples of the labeling substance that can specifically bind to the capture body include a labeled antibody (secondary antibody) that specifically recognizes the capture body.

Although the temperature and time conditions in the labeling step are not particularly limited, for example, a mixture of the complex, the capture body that specifically binds to the tag, and the labeling substance can be incubated at 4 to 60°C, preferably at 25 to 42°C, for 1 second to 24 hours, preferably 1 to 2 hours. During the incubation, the mixture may be allowed to stand, or may be stirred or shaken.

In this embodiment, B/F separation for removing unreacted free components may be performed after mixing of the complex, the capture body and the labeling substance and before signal detection. Examples of the unreacted free component include a free capture body which did not bind to the tag, a free labeling substance which did not bind to the capture body, and the like.

### <Signal detection>

Methods for detecting a signal themselves are known in the field of immunoassay technology. In this embodiment, a measuring method according to the type of signal derived from the labeling substance may be appropriately selected.

For example, when the labeling substance is an enzyme, signals such as light and color generated by reacting a substrate for the enzyme can be measured by using a known apparatus such as a luminometer or a spectrophotometer.

The substrate of the enzyme can be appropriately selected from known substrates according to the type of the enzyme. For example, when ALP is used as the enzyme, examples of the substrate include chemiluminescent substrates such as CDP-Star (registered trademark) (disodium 4-chloro-3-(methoxyspiro[1,2-dioxetane-3,2'-(5'-chloro)tricyclo[3.3.1.13,7]decan]-4-yl)phenyl phosphate) and CSPD (registered trademark) (disodium 3-(4-methoxyspiro[1,2-dioxetane-3,2-(5'-chloro)tricyclo[3.3.1.13,7]decan]-4-yl)phenyl phosphate), and chromogenic substrates such as 5-bromo-4-chloro-3-indolyl phosphate (BCIP), disodium 5-bromo-6-chloro-indolyl phosphate and p-nitrophenyl phosphate. Particularly preferred is CDP-Star (registered trademark). The luminescence of the substrate is preferably detected with a luminometer.

When the labeling substance is a radioactive isotope, radiation as a signal can be measured using a known apparatus such as a scintillation counter. Also, when the labeling substance is a fluorescent substance, fluorescence as a signal can be measured using a known apparatus such as a fluorescence microplate reader. The excitation wavelength and the fluorescence wavelength can be appropriately determined according to the type of fluorescent substance used. In the ELISA method, complex formation and detection thereof may be performed using automated immunoassay system HISCL (Sysmex Corporation).

The phrase "detecting a signal" herein includes qualitatively detecting the presence or absence of a signal, quantifying a signal intensity, and semi-quantitatively detecting the intensity of a signal. Semi-quantitative detection means to show the intensity of the signal in stages such as "no signal generated", "weak", "medium", "strong", and the like.

An aqueous solvent used for diluting the sample and an aqueous solvent for washing to perform B/F separation preferably contain an additive (for example, LIPIDURE (registered trademark)-BL802, or the like) added to prevent labeled cholesterol from adhering to the tube or the like.

The detected signal indicates the amount of cholesterol incorporated into lipoproteins and is an indicator of lipoprotein's ability to uptake cholesterol. This indicator may be represented by optical information such as fluorescence intensity or luminescence intensity, or may be represented by the amount of cholesterol incorporated (such as the number of molecules). The detected signal can be used as a measured value (also referred to as a first measured value) caused by the labeling substance bound to the cholesterol in the first complex.

The measurement of an ability of high-density lipoprotein to uptake cholesterol can be performed, for example, according to the method described in US 2016/0,109,469 A or US 2017/0,315,112 A. US 2016/0,109,469 A or US 2017/0,315,112 A is incorporated herein by reference.

### [Method for quantifying high-density lipoprotein]

The fourth embodiment relates to a method for quantifying high-density lipoprotein (hereinafter sometimes referred to simply as a quantification method).

The monoclonal antibody or antigen-binding fragment described in the section of [Monoclonal antibody or antigen-binding fragment thereof] above can bind not only to high-density lipoprotein incorporating labeled cholesterol but also to high-density lipoprotein not incorporating labeled cholesterol.

Preferably, one of the monoclonal antibody or antigen-binding fragment thereof that forms a complex with the high-density lipoprotein preferably may be a capture antibody (also referred to as a second capture antibody) for capturing the high-density lipoprotein into a solid phase or the like. Also, one of the monoclonal antibody or antigen-binding fragment thereof that forms a complex with the high-density lipoprotein preferably may be a detection antibody for detecting the high-density lipoprotein.

The high-density lipoprotein can be quantified by using a monoclonal antibody or an antigen-binding fragment as a capture antibody and/or a detection antibody. Quantification of high-density lipoprotein can be performed by a known immunoassay. For example, it can be measured by a sandwich ELISA method using the monoclonal or antigen-binding fragment described in the section of [Monoclonal antibody or antigen-binding fragment thereof] above as a capture antibody and a detection antibody.

An epitope of the capture antibody and an epitope of the detection antibody may be the same. Further, since the high-density lipoprotein usually has a plurality of ApoA1 molecules, the epitope of the capture antibody and the epitope of the detection antibody may at least partially overlap or may be different.

Preferably, the capture antibody and the detection antibody used to quantify high-density lipoprotein are each one selected from the group consisting of a monoclonal antibody or antigen-binding fragment thereof that binds to 51st to 110th of the amino acid sequence of SEQ ID NO: 1 and a monoclonal antibody or antigen-binding fragment thereof that binds to 201st to 267th of the amino acid sequence of SEQ ID NO: 1.

More preferably, the capture antibody is a monoclonal antibody or antigen-binding fragment thereof that binds to 51st to 110th of the amino acid sequence of SEQ ID NO: 1, and the detection antibody is a monoclonal antibody or antigen-binding fragment thereof that binds to 201st to 267th of the amino acid sequence of SEQ ID NO: 1. Alternatively, the capture antibody and the detection antibody are both monoclonal antibodies or antigen-binding fragments thereof that bind to 51st to 110th of the amino acid sequence of SEQ ID NO: 1.

Preferably, examples of the monoclonal antibody that binds to 51st to 110th of the amino acid sequence of SEQ ID NO: 1 can include a monoclonal antibody (for example, 8E10 antibody) produced from a hybridoma of accession No. NITE BP-02442 or accession No. NITE BP-02443 or its progeny (including a passage). Preferably, examples of the monoclonal antibody that binds to 201st to 267th of the amino acid sequence of SEQ ID NO: 1 can include a monoclonal antibody (for example, P1A5 antibody) produced from a hybridoma of accession No. NITE BP-02443 or its progeny (including a passage).

The description of the monoclonal or antigen-binding fragment described in the section of [Monoclonal antibody or antigen-binding fragment thereof] above is incorporated in this section.

The quantification of high-density lipoprotein includes a step of mixing high-density lipoprotein, a capture antibody and a detection antibody, thereby forming a complex (also referred to as a second complex), and a step of quantifying the complex.

Data of signal caused by the labeling substance can be used as a measured value (also referred to as a second measured value) of high-density lipoprotein. Alternatively, a measured value of high-density lipoprotein may be generated from the data of signal caused by the labeling substance. For example, when quantitatively detecting the intensity of a signal, the signal intensity or the value calculated from the signal intensity can be used as the measured value. Examples of the value calculated from the signal intensity include a value obtained by subtracting the signal intensity of the negative control sample from the signal intensity of each specimen, a value obtained by dividing the signal intensity of each specimen by the signal intensity of the positive control sample, combinations thereof, and the like. The negative control sample includes samples not containing high-density lipoprotein, and the like. The positive control sample includes samples containing a known amount of high-density lipoprotein (also referred to as a calibrator), and the like.

Also, the measured value of high-density lipoprotein can be calculated by creating a calibration curve from the value of signal intensity of the calibrator and the amount of high-density lipoprotein of the calibrator, and applying the value of the intensity of the signal caused by high-density lipoprotein incorporating the labeled cholesterol of each specimen to the calibration curve. Also, the measured value of high-density lipoprotein can be calculated by obtaining a regression equation from the value of signal intensity of the calibrator, without creating a calibration curve, and applying the value of the intensity of the signal caused by a target substance in each specimen to the regression equation.

The measured value of high-density lipoprotein is, for example, a value reflecting the amount or concentration of high-density lipoprotein per fixed amount of sample.

As the capture antibody and the detection antibody, any of the monoclonal antibody or antigen binding fragment thereof described in the [Monoclonal antibody or anti-binding fragment thereof] and fragments thereof (for example, Fab, F(ab)2, and the like) can be used. Also, immunoglobulin classes and subclasses are not particularly limited.

When the above immunological measurement is performed, an aqueous solvent for diluting the sample and washing to perform B/F separation preferably contains additives (for example, LIPIDURE (registered trademark)-BL802, or the like) added to prevent labeled cholesterol from adhering to the tube or the like.

### [Method 2 for measuring ability of high-density lipoprotein to uptake cholesterol]

The fifth embodiment relates to a second method for measuring an ability of high-density lipoprotein to uptake cholesterol. In this embodiment, the ability of high-density lipoprotein to uptake cholesterol is evaluated based on the first measured value caused by a labeling substance bound to cholesterol acquired by the method described in the [Method 1 for measuring ability of high-density lipoprotein to uptake cholesterol] above and the second measured value of high-density lipoprotein acquired by the method described in the [Method for quantifying high-density lipoprotein] above.

In the method of this embodiment, a first sample is used to measure an ability of high-density lipoprotein to uptake cholesterol, and a second sample is used to quantify high-density lipoprotein. The first and second samples are samples collected from the same subject. The first sample and the second sample may be collected at different times, but are preferably substantially the same time. Here, the phrase "collected at substantially the same time" includes that the first sample and the second sample are collected at the same time, collected on the same day, and collected within several days. Preferably, the first sample and the second sample are collected at the same time. Specifically, a sample collected from the same subject may be divided into two to be a first sample and a second sample.

The method includes measurement step A including a step of mixing high-density lipoprotein in the first sample acquired from a subject with labeled cholesterol to form high-density lipoprotein incorporating the labeled cholesterol, a step of mixing a first capture antibody with the high-density lipoprotein incorporating the labeled cholesterol, thereby forming a first complex of the high-density lipoprotein incorporating the labeled cholesterol and the first capture antibody, and a step of acquiring a first measured value caused by a labeling substance bound to the cholesterol in the first complex.

Also, the method for measuring an ability of high-density lipoprotein to uptake cholesterol of this embodiment includes measurement step B including a step of mixing high-density lipoprotein in the second sample acquired from a same subject as the subject, a second capture antibody, and a detection antibody to form a second complex, and a step of acquiring a second measured value caused by the second complex.

Furthermore, the method for measuring an ability of high-density lipoprotein to uptake cholesterol of this embodiment includes the step of calculating the ability of high-density lipoprotein to uptake cholesterol, based on the first measured value of the second complex acquired in the measurement step A and the second measured value of the second complex acquired in the measurement step B. Specifically, a specific activity can be calculated from the first measured value acquired in the measurement step A and the second measured value of the second complex acquired in the measurement step B. Here, the "specific activity" of the high-density lipoprotein is any value shown in (i) and (ii) below: (i) a value obtained by dividing the first measured value by the second measured value, (ii) a reciprocal of the value obtained in the (i) above.

The measuring method of this embodiment may include the step of outputting a calculated value. The "output" is not particularly limited, and includes, for example, outputting from CPU that has calculated the measurement results to a memory or a monitor, displaying the measurement results on a monitor or the like, transmitting the measurement result to another terminal, printing out the measurement results on paper, and the like.

The method for measuring an ability of high-density lipoprotein to uptake cholesterol follows the method described in the [Method 1 for measuring ability of high-density lipoprotein to uptake cholesterol] above. The method for determining the amount or concentration of high-density lipoprotein follows the method described in the [Method for quantifying high-density lipoprotein] above.

### [Reagent kit 1 for measuring ability of high-density lipoprotein to uptake cholesterol]

A sixth embodiment relates to a reagent kit for measuring an ability of high-density lipoprotein to uptake cholesterol.

The kit includes the monoclonal or antigen-binding fragment described above in the section of [Monoclonal antibody or antigen-binding fragment thereof] above, and labeled cholesterol. When tagged cholesterol is used as the labeled cholesterol, it may further include a capture body to which the labeling substance is bound.

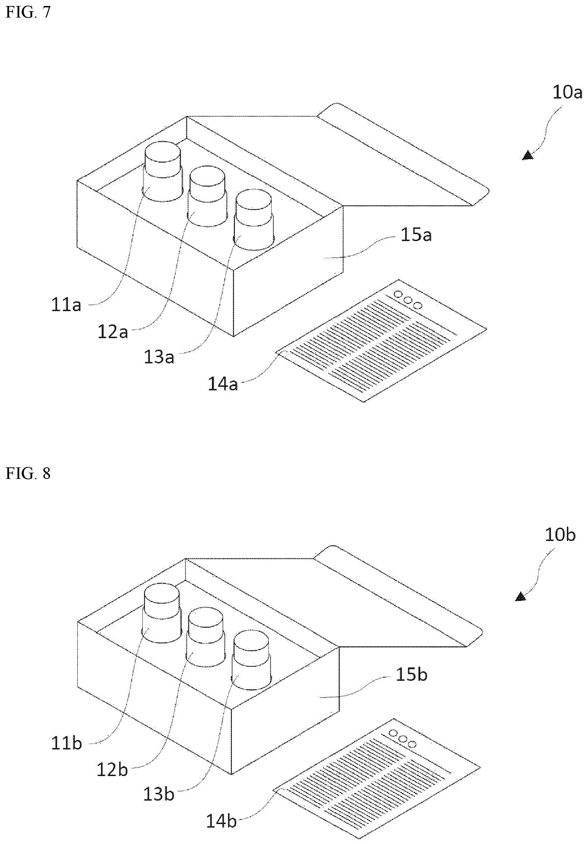

An example of the kit is shown in FIG. 7. Kit 10a contains the monoclonal antibody or antigen-binding fragment described in the [Monoclonal antibody or antigen binding fragment thereof] above, and labeled cholesterol. Examples of the labeled cholesterol can include the labeled cholesterol described in the section of the [Method 1 for measuring ability of high-density lipoprotein to uptake cholesterol] above. These can constitute a kit, for example, in a state stored in a container or the like, in a solution or powder form. In addition, the kit 10a may contain a solvent such as physiological saline or buffer solution (for example, PBS or the like). Moreover, the solvent may contain an additive (for example, LIPIDURE (registered trademark)-BL802, or the like) added to prevent labeled cholesterol from adhering to the tube or the like. FIG. 7 shows the kit 10a including a container 11a storing a monoclonal antibody or antigen-binding fragment, a container 12a storing labeled cholesterol, and a container 13a storing a solvent, as components of the kit. The kit 10a may further include a box 15a containing these containers. In addition, the kit 10a may further include an instruction manual or a sheet 14a describing URL in which the instruction manual can be browsed. The monoclonal antibody or antigen-binding fragment may be immobilized on magnetic beads or plastic beads. Also, instead of the container storage form described in FIG. 7, the monoclonal antibody or antigen-binding fragment may be incorporated into a kit, for example, immobilized on a 96-well microplate. Immobilization of the monoclonal antibody or antigen binding fragment can be performed according to known methods. Moreover, the monoclonal antibody or antigen binding fragment is not directly immobilized on magnetic beads or the like, but may be bound to magnetic beads or the like, for example, via a bond between biotin and avidin (or streptavidin).

### [Reagent kit for quantification of high-density lipoprotein]

A seventh embodiment relates to a reagent kit for quantification of high-density lipoprotein including the monoclonal antibody or antigen-binding fragment described in the section of [Monoclonal antibody or antigen-binding fragment thereof] above.

An example of the kit is shown in FIG. 8. Kit 10b contains the monoclonal antibody or antigen-binding fragment described in the [Monoclonal antibody or antigen binding fragment thereof] above. For example, the monoclonal antibody or antigen-binding fragment can constitute a kit in a state stored in a container or the like, in a solution or powder form. In addition, the kit 10b may contain a solvent such as physiological saline or buffer solution (for example, PBS or the like). Moreover, the solvent may contain an additive (for example, LIPIDURE (registered trademark)-BL802, or the like) added to prevent labeled cholesterol from adhering to the tube or the like. FIG. 7 shows the kit 10b including a container 11b storing a monoclonal antibody or antigen-binding fragment, and a container 13b storing a solvent, as components of the kit. In addition, the kit 10b may include a container 12b storing a detection antibody or the like capable of binding to a labeling substance bound to cholesterol. The reagent kit 10b may further contain a box 15b containing these containers. In addition, the reagent kit 10b may contain an instruction manual or a sheet 14b describing URL in which the instruction manual can be browsed. The monoclonal antibody or antigen-binding fragment may be immobilized on magnetic beads or plastic beads. Also, instead of the container storage form described in FIG. 8, the monoclonal or antigen-binding fragment may be incorporated into a kit, for example, immobilized on a 96-well microplate. Immobilization of the monoclonal antibody or antigen binding fragment can be performed according to known methods. Moreover, the monoclonal antibody or antigen binding fragment is not directly immobilized on magnetic beads or the like, but may be bound to magnetic beads or the like, for example, via a bond between biotin and avidin (or streptavidin).

### [Reagent kit 2 for measuring ability of high-density lipoprotein to uptake cholesterol]

The kit is a reagent kit for measuring a specific activity of high-density lipoprotein. The kit includes the constitution of [Reagent kit 1 for measuring an ability of high-density lipoprotein to uptake cholesterol] and the constitution of [Reagent kit for quantification of high-density lipoprotein] described above.

In addition, as another embodiment of the sixth embodiment and the seventh embodiment, the present invention relates to a use of the monoclonal antibody or antigen-binding fragment described in the section of [Monoclonal antibody or antigen-binding fragment thereof] above, for the manufacture of a reagent kit for functional evaluation of high-density lipoprotein or a kit for quantification of high-density lipoprotein. That is, the monoclonal antibody or antigen-binding fragment of the first embodiment can be used in the manufacture of the reagent kit for functional evaluation of high-density lipoprotein or the kit for quantification of high-density lipoprotein described above. The reagent kit for functional evaluation of high-density lipoprotein or the kit for quantification of high-density lipoprotein is as described above. Examples

Hereinafter, the present invention will be specifically described by way of examples, but the present invention is not to be construed as being limited to the examples.

### Example 1: Preparation of Monoclonal Antibody

### 1. Acquisition of hybridoma

Hybridomas producing monoclonal antibodies were prepared by a mouse iliac lymph node method (JP 4098796 B2). Specifically, inoculation of antigen was performed by injecting an emulsion (antigen concentration: 0.33 mg/mL) obtained by mixing 1 mg/mL of full-length human ApoA1 recombinant protein (Sigma-Aldrich, Inc., product number SRP4693-100UG; derived from E. coli; amino acid sequence is shown in SEQ ID NO: 1) and Freund's complete adjuvant at a volume ratio of 1 : 2 once into the base of the tails of mice (C57B16) at 0.1 mL/mouse. Also, 16 days after the initial inoculation of the antigen, boosting was performed by injecting the above emulsion once again into the base of the tails of the same mice at 0.06 mL/mouse. Furthermore, 4 days after the second antigen inoculation, lymphocytes were isolated from the iliac lymph nodes and fused with cells of myeloma to obtain hybridomas.

### 2. Primary screening

Primary screening of monoclonal antibodies produced by the hybridomas was performed by antigen solid phase ELISA in which two types of positive antigens of human ApoA1 protein used in preparing the hybridomas and an HDL-containing fraction extracted from normal human serum by polyethylene glycol precipitation were immobilized, using a culture supernatant of the hybridomas as a sample. Bovine serum albumin (BSA) was used as a negative control to be immobilized. Primary screening was performed, and wells containing culture supernatants showing reactivity to both of the above two types of positive antigens and low reactivity to negative controls were selected. The antigen solid phase ELISA was performed by the following method. The polyethylene glycol precipitation was performed by the method described in Patent Literature 1 or WO 2016/194825 A.

### <Method>

i. The positive antigen or negative control diluted to 1 µg/mL in PBS was added to each well of 96-well plate at 50 µl/well and incubated at 37°C for 1 hour to immobilize the positive antigen and the negative control in the wells, thereby preparing an antigen-immobilized plate.
ii. Each well was washed twice with 200 µl of PBS and finally the PBS was removed as much as possible.
iii. 200 µl each of 2% BSA-added PBS was added to each well at 200 µl/well and blocked at 25°C for 1 hour.
iv. A culture supernatant of hybridoma containing a monoclonal antibody was diluted 10-fold with StartingBlock (PBS) Blocking buffer (Thermo Fisher SCIENTIFIC/Product No. 37538), added to each well of the antigen-immobilized plate at 50 µl/well, and incubated at 4°C overnight.
v. Each well was washed three times with 200 µl of PBS and finally the PBS was removed as much as possible.
vi. HRP-labeled anti-mouse IgG antibody (Dako/Product No. P0260) was diluted 10,000-fold with StartingBlock (PBS) Blocking buffer, added to each well of the antigen-immobilized plate at 50 µl/well, and incubated at 25°C for 30 minutes.
vii. Each well was washed five times with 200 µl of PBS and finally the PBS was removed as much as possible.
viii. A chemiluminescent substrate solution (SuperSignal ELISA Pico, 37069, Thermo Scientific) was added to each well at 100 µl/well, and the plate was shaken at 600 rpm at 25°C for 2 minutes for luminescence.
ix. The luminescence intensity of each well was measured by a microplate reader (Infinite (registered trademark) F200 Pro, manufactured by TECAN).

### 3. Secondary screening

Secondary screening was performed by antigen solid phase ELISA using culture supernatants of selected hybridomas. For secondary screening, as positive antigens, a total of four types of two types of positive antigens (untreated recombinant ApoA1 or untreated HDL; hereinafter referred to as "untreated recombinant ApoA1", "untreated HDL", respectively) used in the "2. Primary screening" above, and oxidized positive antigens (oxidized recombinant ApoA1 or oxidized HDL; hereinafter referred to as "oxidized recombinant ApoA1" or "oxidized HDL") obtained by oxidizing these positive antigens were used as positive antigens. The negative control was also BSA. Wells containing culture supernatants showing reactivity to the above four types of positive antigens, little difference in reactivity depending on the presence or absence of oxidation treatment, and reaction to negative controls were selected. The antigen solid phase ELISA and the oxidation treatment of antigen were performed by the following method.

### <Method>

i. The positive antigen or negative control diluted to 1 µg/mL in PBS was added to each well of 96-well plate at 50 µl/well and incubated at 37°C for 1 hour to immobilize the positive antigen and the negative control in the wells, thereby preparing an antigen-immobilized plate.
ii. Each well was washed twice with 200 µl of PBS and finally the PBS was removed as much as possible.
iii. In order to prepare two types of oxidized positive antigens, an oxidation treatment solution (PBS containing 1 M hydrogen peroxide, 200 µM sodium nitrite, and 100 µM diethyletriaminepentaacetic acid) was added to the wells on which the respective positive antigens were immobilized. PBS was added to the wells on which two types of positive antigens not to be oxidized were immobilized. The entire plate was incubated at 37°C for 1 hour.
iv. Each well was washed twice with 200 µl of PBS and finally the PBS was removed as much as possible.
v. The luminescence intensity of each well was measured according to iii to ix of the primary screening.

### <Secondary screening result>

Reactivity between the antibody produced by each obtained clone and untreated recombinant ApoA1, untreated HDL, oxidized recombinant ApoA1 or oxidized HDL was confirmed in the "3. Secondary screening" above.

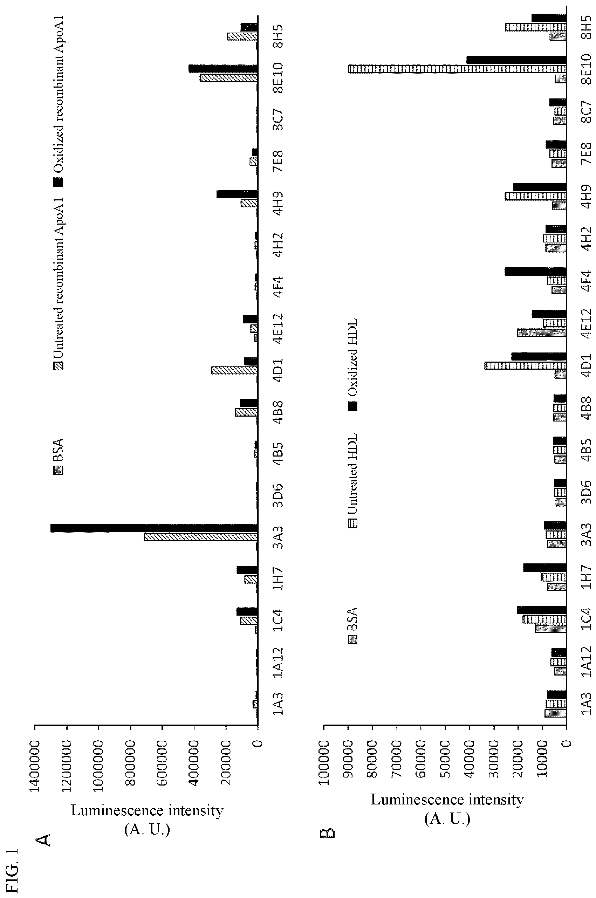

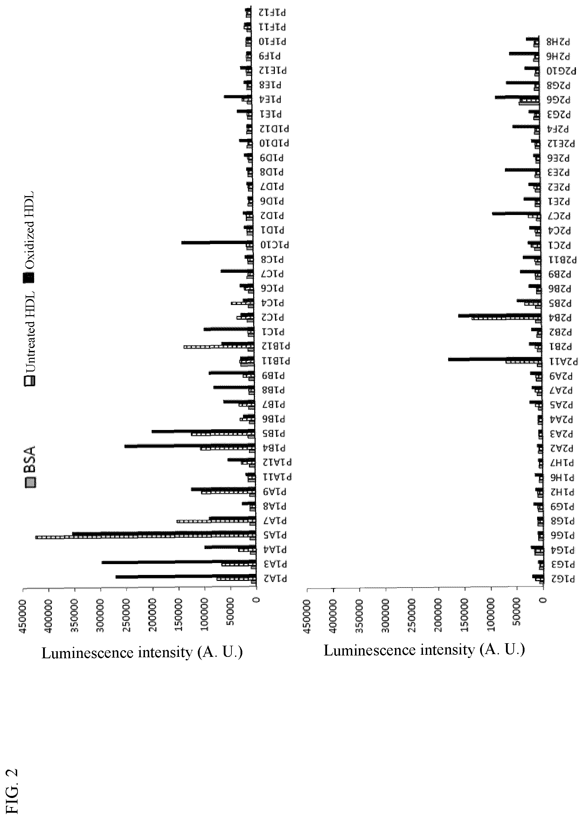

The results are shown in FIGS. 1A, 1B and 2. The horizontal axes of FIG. 1A, FIG. 1B and FIG. 2 show clone names of hybridomas, and the vertical axes show luminescence intensity. (The antibody concentration contained in the supernatant of each hybridoma differs depending on each clone.)

As shown in FIGS. 1A and 1B, the antibody produced by 8E10 clone showed high reactivity to any positive antigen. As shown in FIG. 2, several antibodies including P1A5 and P1A7 showed high reactivity to both untreated HDL and oxidized HDL (Here, confirmation of reactivity of each clone shown in FIG. 2 with recombinant ApoA1 and oxidized recombinant ApoA1 was omitted. It was because each clone shown in FIG. 2 was a clone established using ApoA1 as an antigen, which was considered to naturally bind to ApoA1 if it was confirmed to bind to HDL). Cloning was performed for these three antibodies as follows, and the reactivity was further examined.

### 4. Cloning

The cells in the wells selected in the "3. Secondary screening" above were seeded by limiting dilution. The culture supernatant was again screened according to the method of the "3. Secondary screening" above to acquire three types of desired single clone hybridomas (clone names: 8E10, P1A5 and P1A7). Among these hybridomas, 8E10 and P1A5 were internationally deposited, as identification references "8E10" and "PIA5", respectively, at the Patent Microorganisms Depositary of the National Institute of Technology and Evaluation, located in Room 122, 2-5-8, Kazusakamatari, Kisarazu-shi, Chiba 292-0818 Japan, on March 8, 2017 (identification reference "8E10": accession No. NITE BP-02442, identification reference "PIA5": accession No. NITE BP-02443).

### Example 2: Capture ability of each antibody

Next, BODIPY-labeled cholesterol uptake HDL (labeled cholesterol-HDL) capture property to the monoclonal antibody produced from the hybridoma cloned in Example 1 was evaluated by fluorescence intensity. The monoclonal antibodies evaluated were three types of antibodies (hereinafter referred to as 8E10 antibody, P1A5 antibody and P1A7 antibody, respectively) each derived from clone names 8E10, P1A5 and P1A7, and a commercially available anti-ApoAl antibody (Anti-Apolipoprotein AI/HDL2/HDL3, Mouse-Mono (1C5), SANBIO B.V, product number MON5030; hereinafter, referred to as 1C5 antibody).

HDL to be assayed was crudely purified from human serum by the polyethylene glycol precipitation described above. As human serum, pooled sera (high triglyceride pooled serum: High TG mix) of two kinds of high triglyceride sera (A15, A24) and high triglyceride sera (A16, A18, A19) and pooled sera (normal pooled serum: Normal mix) of three kinds of normal sera (N52, N60, N103) and normal sera (N73, N83, N93) were used. As a negative control, PBS containing LIPIDURE (registered trademark)-BL802 (NOF CORPORATION) was used.

Hereinafter, LIPIDURE (registered trademark)-BL802 was added to a buffer used in an experiment for incorporating labeled cholesterol. Moreover, blocking reagent N101 (NOF CORPORATION) was used for blocking of microplate or the like.

### 1. Evaluation of ability of monoclonal antibody to capture labeled cholesterol-HDL

The three kinds of monoclonal antibodies (8E10 antibody, P1A5 antibody, P1A7 antibody) obtained in Example 1 adjusted to an antibody concentration of 10 µg/mL or the 1C5 antibody were added respectively to each well of a 96-well plate (black plate H for fluorescence measurement, manufactured by Sumitomo Bakelite Co., Ltd.) to prepare an antibody-immobilized plate. Incorporation of labeled cholesterol into HDL was performed according to the method described in Patent Literature 1 or WO 2016/194825 A. Specifically, HDL is mixed with BODIPY-labeled cholesterol (BODIPY trade name TopFluor Cholesterol, CAS No: 878557-19-8, Avanti Polar Lipids, Inc.), and the mixture was incubated with shaking at 37°C for 2 hours to prepare a reaction solution containing labeled cholesterol-HDL. The reaction solution was added to a 96-well plate on which each monoclonal antibody was immobilized, and incubated with shaking at 25°C for 1 hour. After completion of the incubation, the reaction solution was removed and the plate was washed. The plate was washed with 0.01% CHAPS added PBS. A 10 mM methyl-β-cyclodextrin/PBS solution was added, and the mixture was mixed by shaking at 25°C for 1 hour. Then, with respect to the plate, the fluorescence intensity of BODIPY was measured at an excitation/fluorescence wavelength of 485/535 nm, using the fluorescence plate reader (Infinite (registered trademark) 200 Pro, manufactured by TECAN), and the amount of labeled cholesterol-HDL captured was evaluated for the monoclonal antibodies immobilized on the plate.

### 2. Evaluation of ability of monoclonal antibody to capture HDL

The amount of labeled cholesterol-HDL captured by the monoclonal antibody was measured by ELISA using the plate whose fluorescence intensity was measured in the 1. above. Specifically, after measuring the fluorescence intensity in the above 1, the plate was washed to remove 10 mM methyl-β-cyclodextrin. Goat anti-ApoAI antiserum (N assay ApoAI R2 reagent: Nitto Boseki Co., Ltd.) was diluted 1,000-fold with blocking buffer (StartingBlock, Thermo Scientific) and added to each well. After incubation with shaking at 25°C for 1 hour, each well was washed three times with PBS. HRP-labeled rabbit anti-goat IgG antibody was diluted 1,000-fold and added to each well. After incubation with shaking at 25°C for 1 hour, the reaction solution was removed, and each well was washed five times with PBS. A chemiluminescent substrate solution (SuperSignal ELISAPico, 37069, Thermo Scientific) was added to each well at 100 µl/well, and the plate was shaken at 600 rpm at 25°C for 2 minutes for luminescence. The luminescence intensity of each well was measured by a microplate reader (Infinite (registered trademark) F200 Pro, manufactured by TECAN).

### 3. Evaluation of capture ability of 1C5 antibody by presence or absence of oxidation treatment of HDL

The capture abilities of 1C5 antibody were examined by ELISA, using the untreated HDL and the oxidized HDL described in the section of secondary screening in Example 1.

### 4. Results

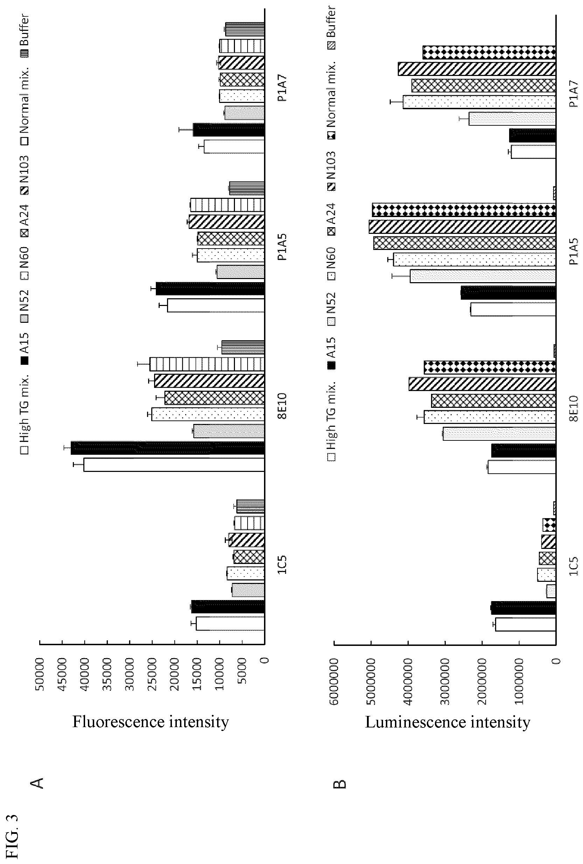

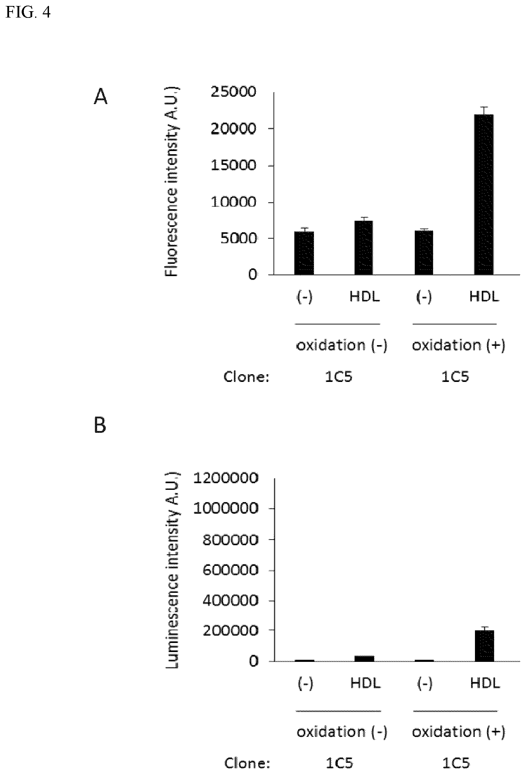

The results are shown in FIGS. 3 and 4. FIG. 3A shows the abilities of 1C5 antibody, 8E10 antibody, P1A5 antibody, and P1A7 antibody to capture labeled cholesterol-HDL. Also, FIG. 3B shows the abilities of these antibodies to capture HDL. Moreover, FIG. 4A shows a comparison result of the abilities of 1C5 antibody to capture labeled cholesterol-HDL between oxidized HDL and non-oxidized HDL. FIG. 4B shows a comparison result of the abilities of 1C5 antibody to capture HDL between oxidized HDL and non-oxidized HDL.

As shown in FIGS. 3A and 3B, when the 1C5 antibody that is a commercially available antibody is evaluated by the fluorescence intensity of labeled cholesterol-HDL, only a fluorescence intensity at the same level as that of a negative control (buffer) was obtained in sera other than A15 and high triglyceride pooled serum (High TG mix). Moreover, even when the capture abilities of 1C5 antibody was evaluated by ELISA, it was confirmed that the capture was insufficient depending on the serum. However, as shown in FIGS. 4A and 4B, the abilities of 1C5 antibody to capture labeled cholesterol-HDL and HDL was improved by oxidizing HDL.

From these results, it was revealed that the commercially available 1C5 antibody has high binding to oxidatively modified HDL and low binding to non-oxidized HDL.

As shown in FIG. 3A, with the 8E10 antibody and P1A5 antibody prepared this time, it was possible to obtain a fluorescence signal even in a serum in which a fluorescence signal was not obtained when the 1C5 antibody was used. In addition, as shown in FIG. 3B, the 8E10 antibody and the P1A5 antibody can detect labeled cholesterol-HDL from all the sera used even by ELISA, and were considered to have higher detection sensitivity than the 1C5 antibody.

On the other hand, the P1A7 antibody was able to capture labeled cholesterol-HDL in all sera and was able to capture labeled cholesterol-HDL to the same degree as the 8E10 antibody (FIG. 3B). However, the fluorescence signal derived from the labeled cholesterol-HDL captured with the P1A7 antibody was lower as compared to when captured with the 8E10 antibody. The HDL captured by the antibody in the present example includes both labeled cholesterol-HDL incorporating BODIPY-labeled cholesterol and HDL not incorporating BODIPY-labeled cholesterol. Therefore, in the ELISA method, a luminescent signal is detected even when either of the two HDLs is captured. The P1A7 antibody was considered not to obtain a BODIPY fluorescence signal because it selectively captured the HDL not incorporating BODIPY-labeled cholesterol.

From the above results, it was considered that the 8E10 antibody and the P1A5 antibody exhibit high binding not only to HDL not incorporating labeled cholesterol but also to labeled cholesterol-HDL.

### Example 3: Determination of binding sites recognized by monoclonal antibodies

The binding sites of 8E10 antibody, P1-A5 antibody, P1-A7 antibody, and 1C5 antibody were determined according to the following method.

A peptide in which a His tag sequence was added to various partial sequences contained in the amino acid sequence of full-length human ApoA1 recombinant protein (derived from E. coli) shown in SEQ ID NO: 1 was prepared, and using this peptide as an antigen, the presence or absence of binding between antibody and antigen was observed by antigen solid phase ELISA shown below. As peptides having partial sequences, a total of 5 types of peptides of 2nd to 60th, 51st to 110th, 101st to 160th, 151st to 210th and 201st to 267th amino acid sequences of the amino acid sequence of SEQ ID NO: 1 were used.

### <Method>

i. The peptides adjusted to 1 ng/mL were added to each well of a 96-well plate.
ii. Each well was washed and then blocked with 2% BSA-added PBS.
iii. Hybridoma culture supernatants of 8E10, P1A5, and P1A7 diluted 10-fold with 2% BSA-added PBS, and 1C5 antibody prepared to 1 µg/ml with 2% BSA-added PBS were added to each well at 50 µl/well. After incubation at 25°C for 1 hour, each well was washed. A 10,000-fold diluted HRP-labeled goat anti-mouse IgG antibody was diluted 1,000-fold, added to each well, and incubated at 25°C for 1 hour. After washing each well, a luminescence substrate was added, and the luminescence intensity was measured using a microplate reader, as in Example 2.

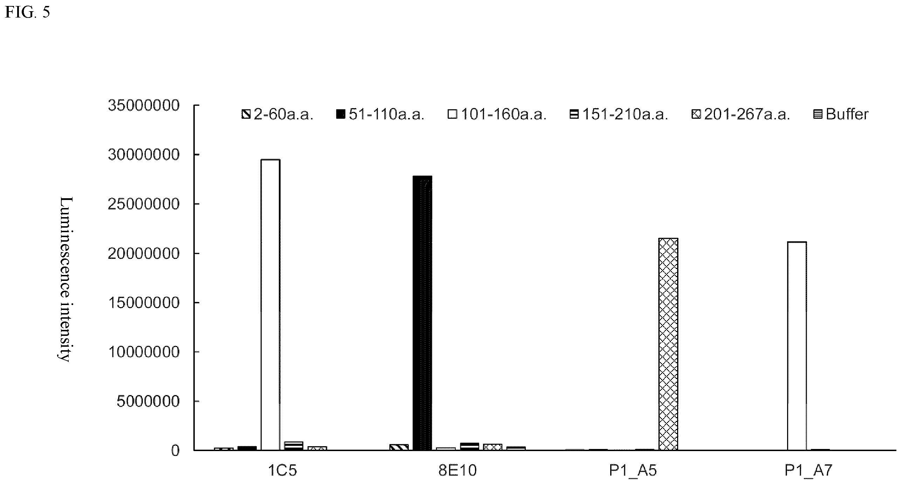

As shown in FIG. 5, the 8E10 antibody bound to the peptide of 51st to 110th of the amino acid sequence of SEQ ID NO: 1, and did not bind to the peptides of other amino acid sequences (2nd to 60th, 101st to 160th, 151st to 210th, 201st to 267th). The P1A5 antibody bound to the peptide of 201st to 267th of the amino acid sequence, and did not bind to the peptides of other amino acid sequences (2nd to 60th, 51st to 110th, 101st to 160th, 151st to 210th). The P1A7 antibody and the 1C5 antibody bound only to the peptide of the 101st to 160th of the amino acid sequence to which the 8E10 antibody and the P1A5 antibody did not bind.

Further, in order to examine the binding site of the 8E10 antibody in more detail, a peptide in which a His tag sequence was added to a partial sequence contained in the range of 51st to 110th of the amino acid sequence of SEQ ID NO: 1 was prepared, and using this peptide as an antigen, the presence or absence of binding between antibody and antigen was observed by antigen solid phase ELISA. As peptides having partial sequences, a total of 5 types of peptides of 51st to 70th, 64th to 83rd, 77th to 96th, and 91st to 110th of the amino acid sequence of SEQ ID NO: 1 were selected. The antigen solid phase ELISA was performed according to the <Method> i to iii of Example 3 except that only the 8E10 antibody was used. Moreover, instead of the 8E10 antibody, the presence or absence of binding to the above peptide was evaluated in the same manner using an anti-His tag antibody.

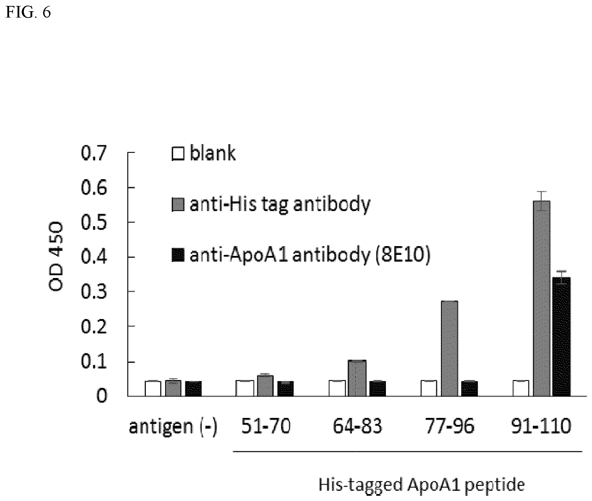

As shown in FIG. 6, the 8E10 antibody showed high binding to the peptide of 91st to 110th of the amino acid sequence of SEQ ID NO: 1. Further, the anti-His tag antibody strongly bound to the peptide of 91st to 110th of the amino acid sequence, and also bound to the peptide of 77th to 96th of the amino acid sequence. From this, it was considered that the binding site of the 8E10 antibody is included in a range of 91st to 110th of the amino acid sequence of SEQ ID NO: 1. Also, it can be seen from FIG. 6 that the binding site of the 8E10 antibody is not included in a range of 51st to 70th, a range of 64th to 83rd, and a range of 77th to 96th of the amino acid sequence of SEQ ID NO: 1.

### Example 4. Examination of combination of antibodies for quantifying levels of high-density lipoprotein

In order to find the combination of supplemental antibody and detection antibody that is most suitable for quantifying levels of high-density lipoprotein (HDL) by ELISA, an ELISA measurement system was constructed using the P1A5 antibody, 8E10 antibody, and polyclonal antibody (goat anti-ApoAI antiserum used in Example 2) as the capture antibody and the detection antibody, using the company's automated immunoassay system HISCL, and the accuracy of the results was compared.

### <Construction of ELISA measurement system>

i. For each antibody, the antibody used as the capture antibody was labeled with biotin according to a known method. The antibody used as the detection antibody was labeled with alkaline phosphatase (ALP) according to a known method.
ii. A buffer of HISCL magnetic beads was removed and washed three times with 0.1% pluronic F68-containing PBS in the same amount as the removed buffer.
iii. The HISCL magnetic beads washed three times with 0.1% pluronic F68-containing PBS were reacted with a biotin-labeled capture antibody to prepare capture antibody-immobilized HISCL magnetic beads for each of the antibodies. The capture antibody-immobilized HISCL magnetic beads were washed three times with 0.1% pluronic F68-containing PBS, and then suspended in the same buffer (used as R2 reagent of HISCL).
iv. HDL fractions were each obtained from specimens according to the polyethylene glycol method described in Patent Literature 1 (hereinafter, referred to as "sample").
v. APBS containing 1 mM cyclodextrin, 0.1% pluronic F68, × 200 Liposome, 1/1000 LIPIDURE (registered trademark)-BL802 + 2% Glycerol was prepared as R1 reagent of HISCL.
vi. Each detection antibody and an alkaline phosphatase-labeled detection antibody were added to R3 buffer of HISCL to prepare R3 reagent.
vii. Each reagent and sample were set in HISCL, and 15 aliquots of each sample were dispensed according to the following protocol (Specimen Nos. 1 to 15) to quantify HDL:
   [Mix 90 µl of R1 reagent and 10 µl of sample] → [42°C, 742 sec] → [Add 30 µl of R2 reagent] → [42°C, 742 sec] → [B/F separation] → [42°C, 568 sec] → [B/F Separation] → [Add 50 µl of R4 reagent, 100 µl of R5 reagent] → [42°C, 305 sec] → Detect luminescence intensity.

The combinations of capture antibody and detection antibody are shown in Table 1.

**[Table 1]**

| Combination of antibodies | Specimen number | | | | | | | | |
|---|---|---|---|---|---|---|---|---|---|
| | No. 1 | No. 2 | No. 3 | No. 4 | No. 5 | No. 6 | No. 7 | No. 8 | No. 9 |
| Capture:P1A5_Detection:PA* | 1961856 | 2185086 | 1996452 | 1259984 | 3528644 | 983787 | 872111 | 794642 | 861432 |
| Capture:P1A5_Detection:8E10 | 1632957 | 2188916 | 1211125 | 1401059 | 1141929 | 2099860 | 1910741 | 1967476 | 1788627 |
| Capture:8E10_Detection:PA* | 1006878 | 1481739 | 1400279 | 647602 | 2967071 | 349834 | 350246 | 325444 | 413736 |
| Capture:8E10_Detection:8E10 | 2556188 | 3121022 | 2258507 | 2665107 | 1947285 | 2987609 | 2638947 | 2649950 | 2690517 |
| Capture:8E10_Detection:P1A5 | 1622561 | 2146425 | 1501816 | 1435315 | 1782791 | 1517510 | 1651165 | 1586811 | 1394231 |

| Combination of antibodies | Specimen number | | | | | | No. 1∼No. 15 | | |
|---|---|---|---|---|---|---|---|---|---|
| | No. 10 | No. 11 | No. 12 | No. 13 | No. 14 | No. 15 | Average | SD | CV% |
| Capture:P1A5_Detection:PA* | 776202 | 605097 | 994938 | 913404 | 827314 | 728799 | 1285983 | 799655 | 62 |
| Capture:P1A5_Detection:8E10 | 1768959 | 1879592 | 1795706 | 1696938 | 1857146 | 1743305 | 1738956 | 294839 | 17 |
| Capture:8E10_Detection:PA* | 300019 | 487717 | 287068 | 278745 | 347384 | 419668 | 737562 | 733874 | 99 |
| Capture:8E10_Detection:8E10 | 2630231 | 2726826 | 2365824 | 2401302 | 2548906 | 2513379 | 2580107 | 280079 | 11 |
| Capture:8E10_Detection:P1A5 | 1396535 | 1709376 | 1397553 | 1283489 | 1522347 | 1561829 | 1567317 | 208256 | 13 |

### <Results>

The results of quantification, mean value (Average), standard deviation (SD) and variance (CV%) are shown in Table 1.

In a system using the P1A5 antibody or 8E10 antibody as the capture antibody and the polyclonal antibody as the detection antibody, the CVs were 62% and 99%, respectively, which revealed that the variation between measurements was large and the measurement results were not stable. On the other hand, when the P1A5 antibody was used as the capture antibody and the 8E10 antibody was used as the detection antibody, the CV was 17%, which revealed that the variation between measurements was small. When the 8E10 antibody was used as the capture antibody and the 8E10 antibody or P1A5 antibody was used as the detection antibody, the CVs were 11% and 13%, respectively, which revealed that the variation between measurements was small.

From the above results, when quantifying HDL, it was considered preferable to use 8E10 antibody and/or P1A5 antibody for the capture antibody and the detection antibody.

### Reference Signs List

10a reagent kit for functional evaluation of high-density lipoprotein
11a container storing monoclonal or antigen-binding fragment
12a container storing labeled cholesterol
13a container storing solvent
14a instruction manual or sheet describing url in which instruction manual can be browsed
15a box containing the above container
10b reagent kit for quantification of high-density lipoprotein
11b container storing monoclonal or antigen-binding fragment
12b container storing capture antibody for detection of labeling substance bound to cholesterol
13b container storing solvent
14b instruction manual or sheet describing url in which instruction manual can be browsed
15b box containing the above container

### [Sequence Listing]

New P18-038WO_PCT_anti-ApoA1 antibody_20180330_112947_12.txt

## Claims

1. A monoclonal antibody or antigen-binding fragment, **characterized in that**
the monoclonal antibody or antigen-binding fragment is capable of binding to a peptide consisting of 51st to 110th amino acid sequence or a peptide consisting of 201st to 267th amino acid sequence of an amino acid sequence of SEQ ID NO: 1, and
the monoclonal antibody or antigen-binding fragment is capable of binding to high-density lipoproteins of (1) to (3) below;
(1) high-density lipoprotein that is oxidatively modified,
(2) high-density lipoprotein that is not oxidatively modified, and
(3) high-density lipoprotein incorporating labeled cholesterol represented by formula (I):

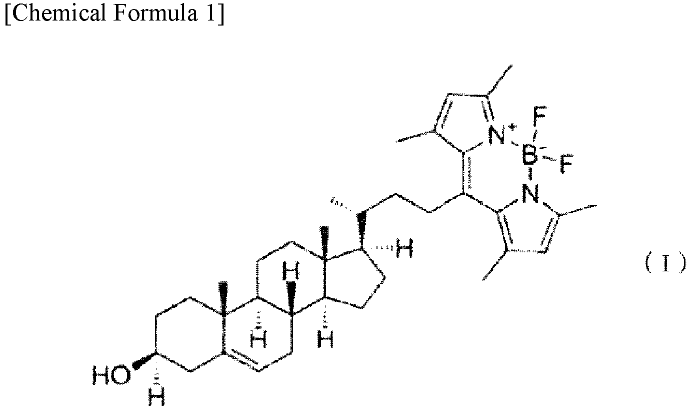

2. The monoclonal antibody or antigen-binding fragment thereof according to claim 1, **characterized in that** the monoclonal antibody or antigen-binding fragment binds to a peptide consisting of 91st to 110th amino acid sequence of the amino acid sequence of SEQ ID NO: 1 and does not bind to a range of 101st to 160th of the amino acid sequence of SEQ ID NO: 1.

3. The monoclonal antibody or antigen-binding fragment thereof according to claim 1 or 2, **characterized in that** the monoclonal antibody or antigen-binding fragment binds to non-denatured high-density lipoprotein present in a sample under non-denatured conditions.

4. The monoclonal antibody or antigen-binding fragment thereof according to any one of claims 1 to 3, **characterized in that** the monoclonal antibody or antigen-binding fragment containing HCDR1, HCDR2, HCDR3, LCDR1, LCDR2 and LCDR3 comprising amino acid sequences that are respectively same as amino acid sequences of HCDR1, HCDR2, HCDR3, LCDR1, LCDR2 and LCDR3 of monoclonal antibody produced from a hybridoma of accession No. NITE BP-02442 or accession No. NITE BP-02443 or its progeny,
wherein HCDR represents a heavy chain complementarity determining region and LCDR represents a light chain complementarity determining region.

5. The monoclonal antibody or antigen-binding fragment thereof according to any one of claims 1 to 3, **characterized in that** the monoclonal antibody contains a heavy chain variable region and a light chain variable region comprising amino acid sequences that are respectively same as amino acid sequences of a heavy chain variable region and a light chain variable region of monoclonal antibodies produced from a hybridoma of accession No. NITE BP-02442 or accession No. NITE BP-02443 or its progeny.

6. The monoclonal antibody or antigen-binding fragment thereof according to any one of claims 1 to 3, **characterized in that** the monoclonal antibody contains an amino acid sequence that is same as an amino acid sequence of a monoclonal antibody or an antigen-binding fragment thereof produced from a hybridoma of accession No. NITE BP-02442 or accession No. NITE BP-02443 or its progeny.

7. A monoclonal antibody or antigen-binding fragment thereof produced from a hybridoma of accession No. NITE BP-02442 or accession No. NITE BP-02443 or its progeny.

8. A hybridoma of accession number of accession No. NITE BP-02442 or accession No. NITE BP-02443 or its progeny.

9. A method for measuring an ability of high-density lipoprotein to uptake cholesterol, comprising
a step of mixing the monoclonal antibody or antigen-binding fragment thereof according to any one of claims 1 to 7, a labeled cholesterol containing a labeling substance and high-density lipoprotein, thereby forming a complex containing the high-density lipoprotein incorporating the labeled cholesterol and the monoclonal antibody or antigen-binding fragment thereof, and
a step of detecting a signal caused by the labeling substance in the complex.

10. The method according to claim 9, wherein the labeled cholesterol is labeled cholesterol represented by formula (I):

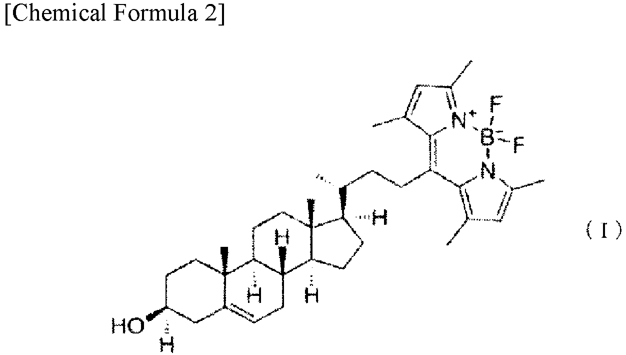

11. A reagent kit for functional evaluation of high-density lipoprotein, comprising
the monoclonal antibody or antigen-binding fragment thereof according to any one of claims 1 to 7, and
a labeled cholesterol.

12. The kit according to claim 11, further comprising an antibody capable of binding to the labeled cholesterol.

13. The kit according to claim 11 or 12, wherein the labeled cholesterol is labeled cholesterol represented by formula (I):

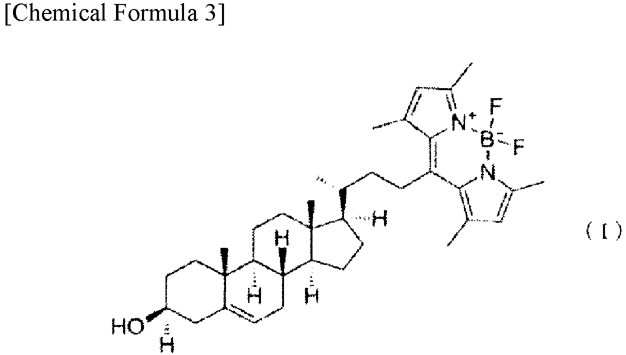

14. A method for quantifying high-density lipoprotein, comprising
a step of mixing high-density lipoprotein, a capture antibody, and a detection antibody to form a complex; and
a step of quantifying the complex,
wherein
each of the capture antibody and the detection antibody is one selected from the group consisting of a monoclonal antibody or antigen-binding fragment thereof that binds to 51st to 110th of an amino acid sequence of SEQ ID NO: 1 and a monoclonal antibody or antigen-binding fragment thereof that binds to 201st to 267th of the amino acid sequence of SEQ ID NO: 1.

15. The quantitative method according to claim 14, wherein
the capture antibody is a monoclonal antibody or antigen-binding fragment thereof that binds to 51st to 110th of the amino acid sequence of SEQ ID NO: 1, and
the detection antibody is a monoclonal antibody or antigen-binding fragment thereof that binds to 201st to 267th of the amino acid sequence of SEQ ID NO: 1.

16. The quantitative method according to claim 14, wherein the capture antibody and the detection antibody are antibodies that bind to a same epitope.

17. The quantitative method according to claim 14, wherein the capture antibody and the detection antibody are monoclonal antibodies or antigen-binding fragments thereof that bind to 51st to 110th of the amino acid sequence of SEQ ID NO: 1.

18. The quantitative method according to any one of claims 14 to 17, wherein the capture antibody and the detection antibody bind to both of high-density lipoprotein that is oxidatively modified and high-density lipoprotein that is not oxidatively modified.

19. The quantitative method according to any one of claims 14 to 18, wherein the capture antibody and the detection antibody bind to high-density lipoprotein incorporating labeled cholesterol represented by formula (I):

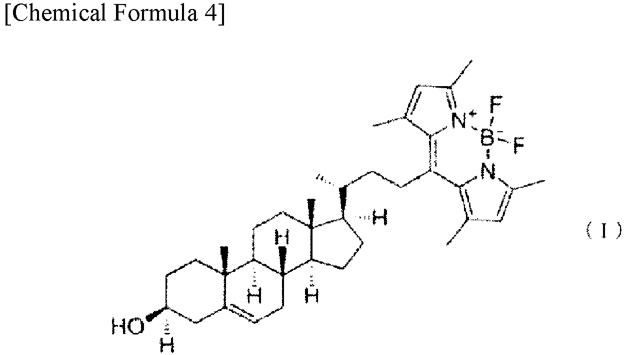

20. The quantitative method according to any one of claims 14 to 19, wherein the detection antibody contains a labeling substance, and
a signal derived from the labeling substance contained in the complex is detected in the step of quantifying the complex.

21. The quantitative method according to any one of claims 14 to 19, wherein the capture antibody is immobilized on a solid phase.

22. A method for measuring an ability of high-density lipoprotein to uptake cholesterol, comprising:
a step of mixing high-density lipoprotein in a first sample acquired from a subject with labeled cholesterol to form high-density lipoprotein incorporating the labeled cholesterol,
a step of mixing a first capture antibody with the high-density lipoprotein incorporating the labeled cholesterol, thereby forming a first complex of the high-density lipoprotein incorporating the labeled cholesterol and the first capture antibody, and
a step of acquiring a first measured value caused by a labeling substance in the first complex; and
a step of mixing high-density lipoprotein in a second sample acquired from a same subject as the subject, a second capture antibody, and a detection antibody to form a second complex,
a step of acquiring a second measured value caused by the second complex, and
a step of calculating the ability of the high-density lipoprotein to uptake cholesterol, based on the first measured value and the second measured value,
wherein,
the first capture antibody is the monoclonal antibody or antigen-binding fragment thereof according to any one of claims 1 to 7, and
each of the second capture antibody and the detection antibody is a monoclonal antibody or antigen-binding fragment thereof that binds to 51st to 110th of an amino acid sequence of SEQ ID NO: 1 or a monoclonal antibody or antigen-binding fragment thereof that binds to 201st to 267th of the amino acid sequence of SEQ ID NO: 1.

23. The method for measuring an ability of high-density lipoprotein to uptake cholesterol according to claim 22, further comprising a step of outputting a calculation result after the step of calculating.

24. The method for measuring an ability of high-density lipoprotein to uptake cholesterol according to claim 23, wherein (i) a value obtained by dividing the first measured value by the second measured value and/or (ii) a reciprocal of the value obtained in (i) is calculated in the step of calculating.
